# EUROPEAN PATENT APPLICATION

(11) **EP 2 685 265 A2**
(43) Date of publication of application: **15.01.2014**
(21) Application number: 13175923.5
(22) Date of filing: 10.07.2013
(51) Int. Cl.: G01N 35/00, B01L 3/00

(54) **Test device and control method thereof**

(30) Priority: 11.07.2012 KR 20120075686
(71) Applicant: Samsung Electronics Co., Ltd, Gyeonggi-do 443-742 (KR)
(72) Inventor: Kim, Jong Cheol, Seoul (KR)
(74) Representative: Land, Addick Adrianus Gosling

(57) **Abstract**

A test device includes a microfluidic device to conduct a test for biomolecules and controlled to prevent errors in the test, and a control method thereof includes mounting a microfluidic device on a tray, positioning a rotary drive unit at a center of the microfluidic device, and moving the rotary drive unit up and down at least once such that the microfluidic device is seated on the rotary drive unit.

## Description

The present invention relates generally to a test device, and more particularly, to a test device having a microfluidic device to conduct a test for biomolecules and a control method thereof.

Microfluidic devices are used to perform biological or chemical reactions by manipulating small amounts of fluid.

A microfluidic structure provided in a microfluidic device to perform an independent function generally includes a chamber to accommodate a fluid, a channel allowing the fluid to flow therethrough, and a member to regulate the flow of the fluid. The chamber, channel, and valve may be disposed in the platform in a variety of configurations. The microfluidic structure may be realized through various combinations of the chamber, channel, and valve. A device fabricated by disposing such a microfluidic structure on a chip-shaped substrate to perform tests involving immune serum reaction or biochemical reaction on a small chip and multi-step processing and manipulation, is referred to as a lab-on-a chip.

To transfer a fluid in a microfluidic structure, driving pressure is needed. Capillary pressure or pressure generated by a separate pump may be used as the driving pressure. Recently, a disc type microfluidic device which has a microfluidic structure arranged on a disk-shaped platform and moves a fluid using centrifugal force to perform a series of operations has been proposed. This device is referred to as Lab CD or Lab-on a CD.

A test device having a microfluidic device generally includes a rotary drive unit to drive the microfluidic device to perform a test. In conventional cases, when the microfluidic device is mounted to the test device, the rotary drive unit determines if the microfluidic device has been mounted and performs a test. However, when the microfluidic device includes a magnetic body, attraction between a magnet included in a detection module and the magnetic body of the microfluidic device may prevent the microfluidic device from being normally seated in the rotary drive unit. As a result, the microfluidic device may not normally rotate, and thereby the test may fail.

Accordingly, there is a need to address the above-mentioned problems and/or disadvantages and to offer at least the advantages described below.

An aspect of the present invention is to provide a test device and an improved control method thereof which allow the microfluidic device to be normally seated.

Additional aspects of the invention will be set forth in part in the following description.

In accordance with one exemplary aspect of the present invention, a control method of a test device includes mounting a microfluidic device on a tray, upon mounting the microfluidic device on the tray, positioning a rotary drive unit at a center of the microfluidic device, and moving the rotary drive unit up and down at least once such that the microfluidic device is seated on the rotary drive unit.

In accordance with another aspect of the present invention, a test device includes a housing forming an external appearance of the test device, a tray, a microfluidic device being loaded onto the tray, a rotary drive unit coupled to a center of the microfluidic device and configured to move in a first movement toward a position at which the tray is placed and a second movement in a direction opposite to the first movement, an opening-closing drive unit configured to move the rotary drive unit to perform the first movement and the second movement at least once, and a controller to control the opening-closing drive unit to initiate the rotary drive unit to perform the first movement and the second movement at least once when the microfluidic device is loaded onto the tray such that the microfluidic device is accommodated at a predetermined position..

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a perspective view schematically illustrating a structure of a microfluidic device according to an exemplary embodiment of the present invention;
FIGS. 2A, 2B and2C are plan views illustrating the structure of the microfluidic device according to the exemplary embodiment in detail;
FIG. 3 is a graph schematically illustrating the rotational speed of a platform during respective fluid transfer operations in the microfluidic device according to the exemplary embodiment;
FIGS. 4A, 4B, 4C, 4D, and 4E are plan views illustrating flow of a fluid in the microfluidic device according to the exemplary embodiment;
FIG. 5 is a function block diagram of a test device according to one exemplary embodiment;
FIG. 6 is a plan view illustrating a tray in a closed state according to the exemplary embodiment of the present invention, which is in an open state;
FIG. 7 is a plan view illustrating the tray according to the exemplary embodiment of the present invention, which is in a closed state;
FIG. 8 is a plan view illustrating a bottom surface of an upper housing according to the exemplary embodiment of the present invention;
FIG. 9 is a view illustrating the microfluidic device according to one embodiment of the present invention, which is abnormally coupled to a rotary drive unit and a clamper;
FIG. 10 is a view illustrating the microfluidic device according to the embodiment of the present invention, which is separated from the rotary drive unit and the clamper;
FIG. 11 is a view illustrating the microfluidic device according to the embodiment of the present invention, which is normally coupled to a rotary drive unit and a clamper;
FIG. 12 is a flowchart illustrating a control method of a test device according to an exemplary embodiment;
FIG. 13 is a flowchart illustrating a control method of a test device according to another embodiment of the present invention;
FIG. 14 is a flowchart showing a control method of a test device according to another embodiment of the present invention;
FIG. 15 is a block diagram showing the configuration of a test device according to one embodiment of the present invention;
FIG. 16 is a conceptual side view illustrating the configuration of the test device according to the one embodiment of the present invention;
FIGS. 17 and 18 are views illustrating radial movement of a detection module according to the one embodiment of the present invention, as seen from the top;
FIGS. 19, 20 and 21 are views illustrating movement of a detection object to a position facing the light receiving element of the detection module by movement of the detection module and rotation of the microfluidic device in the test device according to the one embodiment of the present invention, as viewed from the top;
FIG. 22 is a block diagram illustrating a configuration of a test device according to another embodiment of the present invention of the present invention;
FIG. 23 is a conceptual side view illustrating the configuration of the test device of FIG. 22;
FIGS. 24 and 25 are views illustrating radial movement of a detection module according to the another embodiment of the present invention;
FIGS. 26, 27 and 28 are views illustrating movement of a detection object to a position facing a light receiving element of a detection module by movement of the detection module and rotation of the microfluidic device in a test device according to the another embodiment of the present invention;
FIG. 29 is a flowchart illustrating a control method of a test device according to one embodiment of the present invention;
FIG. 30 is a flowchart illustrating a control method of a test device according to another embodiment of the present invention;
FIG. 31 is a view schematically illustrating test paper used as an example of the detection object of a test device according to an embodiment of the present invention; and
FIG. 32 is a flowchart illustrating a control method of a test device according to the embodiment of the present invention to capture an image of a detection object maintaining a uniform brightness of the test paper.

Throughout the drawings, the same or like drawing reference numerals will be understood to refer to the same or like elements, features and structures.

### DETAILED DESCRIPTION OF EXEMPLARY EMBODIMENTS

Reference will now be made in detail to the non-limiting embodiments of the present invention, examples of which are illustrated in the accompanying drawings, wherein like reference numerals refer to like elements throughout. This invention may however, be embodied in many different forms and should not be construed as limited to the exemplary embodiments set forth herein. Rather, the disclosed embodiments are provided so that this invention will be thorough and complete, and will fully convey the scope to those skilled in the art.

A microfluidic device according to an exemplary embodiment of the present invention will first be described and then a description will be given of a test device to detect a result of a biochemical reaction occurring on a detection object of the microfluidic device. An example of the test device is a blood testing device.

For the test device, a structure and control method of seating the microfluidic device in the test device will first be described and a detection module to detect the detection object positioned at various radii on the microfluidic device will be described, and finally a description will be given of a method of detecting the detection object while maintaining a uniform brightness of the detection object to reduce variation in detection results of the detection object.

### <Microfluidic Device>

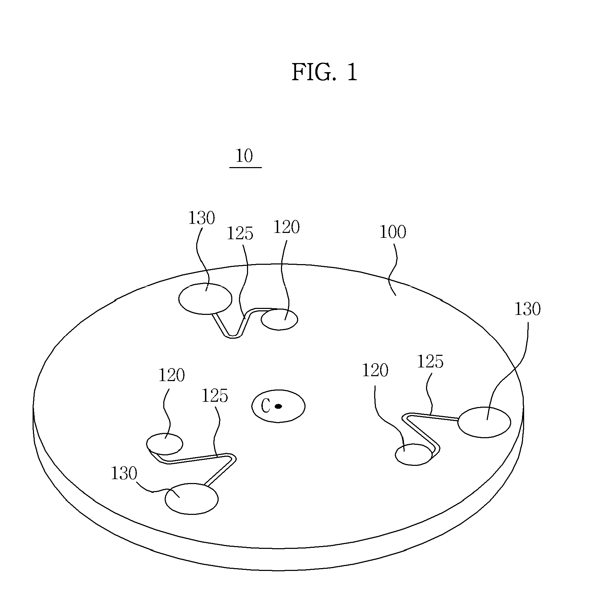

A microfluidic device according to an exemplary embodiment of the present invention will be described below with reference to FIGS. 1 to 4.

FIG. 1 is a perspective view schematically illustrating the microfluidic device according to an exemplary embodiment of the present invention.

Referring to FIG. 1, the microfluidic device 10 according to the illustrated embodiment includes a platform 100 on which a microfluidic structure is formed and a microfluidic structure formed thereon.

The microfluidic structure includes a plurality of chambers to accommodate a fluid and a channel to connect the chambers.

Here, the microfluidic structure is not limited to a structure with a specific shape, but comprehensively refers to structures, including the channel connecting the chambers to each other, formed on the microfluidic device, especially on the platform of the microfluidic device to allow flow of fluid. The microfluidic structure may perform different functions depending on arrangements of the chambers and the channel and the kind of the fluid accommodated in the chambers or flowing along the channel.

The platform 100 may be made of various materials including plastics such as polymethylmethacrylate (PMMA), polydimethylsiloxane (PDMS), polycarbonate (PC), polypropylene, polyvinyl alcohol and polyethylene, glass, mica, silica and silicon (in the form of a wafer) which are easy to work with and whose surfaces are biologically inactive. The above materials are simply examples of materials usable for the platform 100, and embodiments of the present invention are not limited thereto. Any material having proper chemical and biological stability, optical transparency and mechanical workability may be used as a material of the platform 100.

The platform 100 may be formed in multiple layers of plates. A space to accommodate a fluid in the platform 100 and a channel allowing the fluid to flow therethrough may be provided by forming intaglio structures corresponding to the microfluidic structures such as the chambers and the channel on the contact surfaces of two plates and joining the plates. Joining two plates may be implemented using various techniques such as binding with an adhesive agent or a double-sided adhesive tape, ultrasonic welding and laser welding.

The illustrated embodiment of FIG. 1 employs a circular plate-shaped disc type platform 100, but the platform 100 used in the illustrated embodiment may have the shape of a whole circular plate which is rotatable or a sector rotatable in a rotatable frame when seated thereon, or it may have any polygonal shape so long as such shape is rotatable.

A platform 100 may be provided with one test unit, which may include cambers 120 and 130 along with channel 125. However, for faster throughput at lower cost, the platform 100 may be divided into a plurality of sections and each section may be provided with a microfluidic structure which is operable independently of other microfluidic structures. The microfluidic structures may be assigned to different tests and perform several tests at the same time. Alternatively, a plurality of test units to perform the same test may be provided. For convenience of description of the illustrated embodiment, it will be hereinafter assumed that a chamber to receive a sample from a sample supply chamber and a channel connected to the chamber form a unit A different sample supply chamber means a different unit.

Since the microfluidic device 10 according to the illustrated embodiment causes the fluid to move using centrifugal force, the chamber 130 to receive the fluid is disposed at a position more distant from the center of the platform 100 than the position of the chamber 120 to supply the fluid, as shown in FIG. 1.

The two chambers are connected by the channel 125, and in the microfluidic device 10 of the illustrated embodiment, a siphon channel may be used as the channel 125 to control the fluid flowing therethrough.

The siphon channel refers to a channel which causes a fluid to move using pressure difference. In the microfluidic device 10, the flow of the fluid through the siphon channel is controlled using capillary pressure that forces the fluid to move up through a tube having a very small cross-sectional area and centrifugal force generated by rotation of the platform 100. That is, the inlet of the siphon channel having a very small cross-sectional area is connected to the chamber in which the fluid is accommodated, and the outlet of the siphon channel is connected to another chamber to which the fluid is transferred. Here, a point at which the siphon channel is bent, i.e., the highest point of the siphon channel should be higher than the level of the fluid accommodated in the chamber. Also, since the fluid positioned closer to the outside of the platform 100 than the inlet of the siphon channel is not transferred, the position of the inlet of the siphon channel depends on the amount of the fluid to be transferred. When the siphon channel is filled with the fluid by the capillary pressure of the siphon channel, the fluid filling the siphon channel is transferred to the next chamber by centrifugal force.

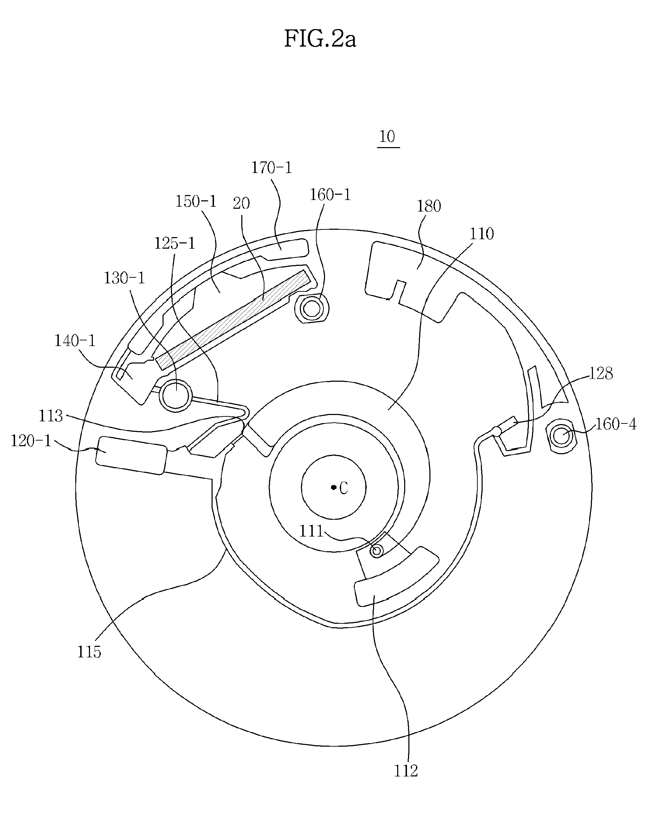

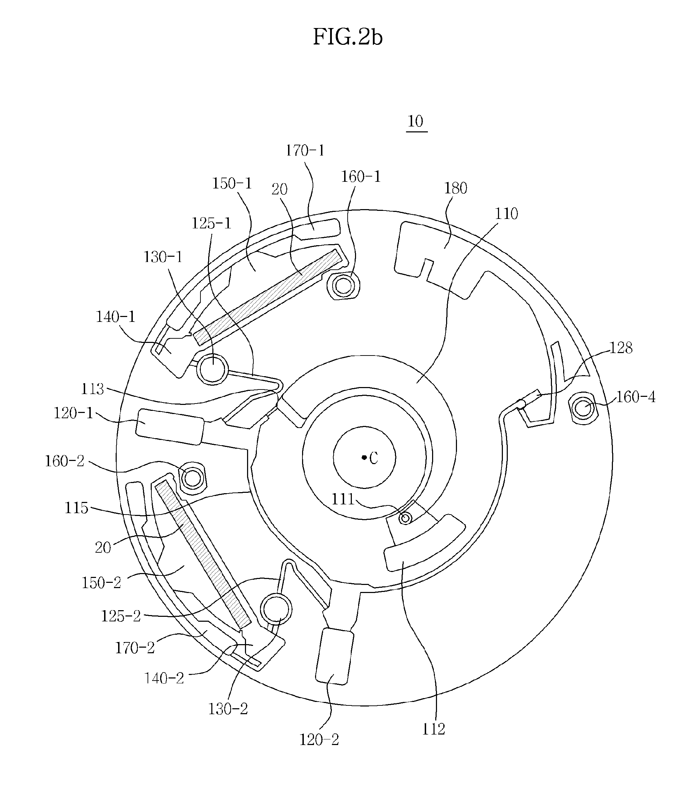

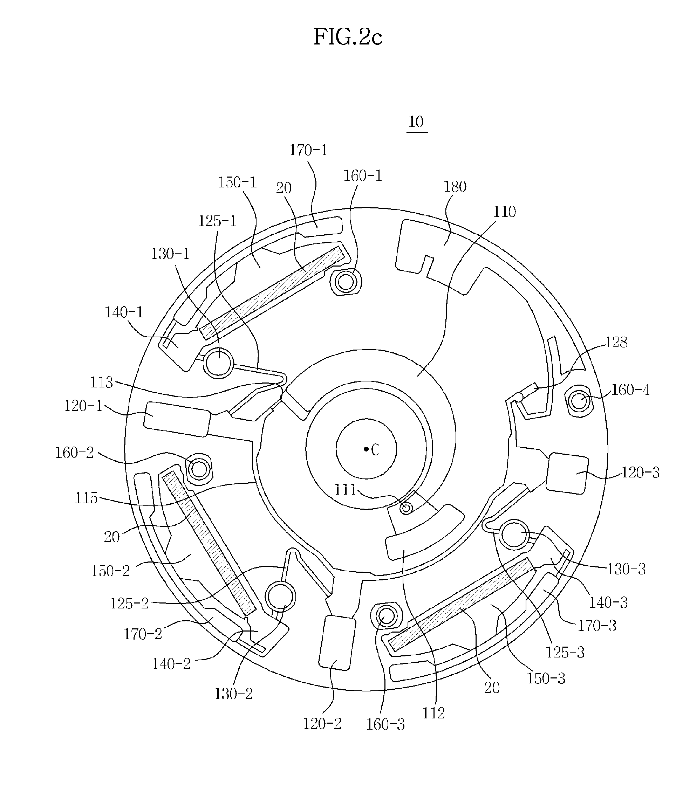

Hereinafter, the structure and operation of the microfluidic device according to the illustrated embodiment will be described in detail with reference to FIGS. 2 to 4.

FIGS. 2A to 2C show plan views of the structure of the microfluidic device according to the exemplary embodiment in detail. Hereinafter, the structure of the microfluidic device 10 will be described in detail with reference to FIGS. 2A to 2C.

As described above, the platform 100 may be formed in various shapes including circles, circular sectors and polygons, and in the illustrated embodiment, the platform 100 has a circular shape. Also, as shown in FIGS. 2A to 2C, at least one first chamber may be connected to a distribution channel. For convenience of description, in the illustrated embodiment, it will be assumed that three first chambers 120, namely, chambers 120-1, 120-2 and 120-3 are connected in parallel to the distribution channel 115 and three second chambers 130-1,130-2 and 130-3 are connected to the respective first chambers 120, as shown in FIG. 2C.

The sample supply chamber 110 is formed at a position close to the center of rotation C to accommodate a sample supplied from the outside. The sample supply chamber 110 accommodates a sample in a fluidic state, and in the illustrated embodiment, the sample supplied is blood.

A sample introduction inlet 111 is provided at one side of the sample supply chamber 110, and an instrument such as a pipette may be used to introduce blood into the sample supply chamber 110 through the sample introduction inlet 111. Blood may be spilled near the sample introduction inlet 111 during the introduction of blood, or the blood may flow backward through the sample introduction inlet 111 during rotation of the platform 100. To prevent the microfluidic device 10 from being contaminated by such events, a backflow receiving chamber 112 may be formed on the upper surface of the microfluidic device 10 adjacent to the sample introduction inlet 111 to accommodate the blood spilled during introduction thereof or the blood flowing backward.

In another embodiment to prevent backflow of the blood introduced into the sample supply chamber 110, a structure to function as a capillary valve (not shown) to allow passage of a sample only when a pressure greater than or equal to a predetermined level is applied may be formed in the sample supply chamber 110.

In a further embodiment to prevent backflow of the blood introduced into the sample supply chamber 110, a rib-shaped backflow prevention device (not shown) may be formed in the sample supply chamber 110. Arranging the backflow prevention device in a direction crossing the direction of flow of the sample from the sample introduction inlet 111 to the sample discharge outlet may produce resistance to flow of the sample, thereby preventing the sample from flowing toward the sample introduction inlet 111.

The sample supply chamber 110 may be formed to have a width gradually increasing from the sample introduction inlet 111 to the sample discharge outlet 113 in order to facilitate discharge of the sample accommodated therein through the sample discharge outlet 113.

The sample discharge outlet 113 of the sample supply chamber 110 is connected to the distribution channel 115 formed on the platform 100 in the circumferential direction of the platform 100, and the distribution channel 115 is sequentially connected to the "1-1 "th chamber 120-1, the "1-2"th chamber 120-2 and the "1-3"th chamber 120-3 as it extends counterclockwise. A Quality Control (QC) chamber 128 to indicate completion of supply of the sample and an excess chamber 180 to accommodate an excess portion of the sample remaining after supply of the sample may be connected to the end of the distribution channel 115.

The first chambers 120 may accommodate the sample supplied from the sample supply chamber 110 and cause the sample to separate into supernatant and sediment through centrifugal force. Since the sample used in the illustrated embodiment is blood, the blood may separate into the supernatant including serum and plasma and sediment including corpuscles in the first chambers 120.

Each of the first chambers 120-1, 120-2 and 120-3 is connected to a corresponding one of the siphon channels 125-1,125-2 and 125-3. As described above, the highest points of the siphon channels 125 should be higher than the highest level of the fluid accommodated in the first chambers 120, and to secure a difference in height, the "1-2"th chamber 120-2 is positioned on a circumference more distant from the center of rotation C or a circumference of a larger radius than the circumference on which the "1-1 "th chamber 120-1 is positioned, and the "1-3"th chamber 120-3 is positioned on a circumference more distant from the center of rotation C or a circumference of a larger radius than the circumference on which the "1-2"th chamber 120-2 is positioned.

In this arrangement, first chambers 120 positioned farther away from the sample discharge outlet 113 have a shorter length in a radial direction. Accordingly, if the first chambers 120 are set to have the same volume, one of the first chambers 120 positioned farther away from the sample discharge outlet 113 has a larger width in a circumferential direction.

As described above, the positions at which the inlets of the siphon channels 125-1,125-2 and 125-3 meet the outlets of the first chambers 120-1, 120-2 and 120-3 change depending on the amount of fluid to be transferred. Thus, if the sample is blood, as in the illustrated embodiment, a test is often performed only on the supernatant, and therefore the outlets of the first chambers 120 may be arranged at upper portions of the first chambers 120, at which the supernatant is positioned, above the middle portions of the first chambers 120. Also, protrusions (not shown) connected to the siphon channels 125-1, 125-2 and 125-3 may be provided at the outlets of the first chambers 120 to facilitate flow of the fluid. This is simply an embodiment provided for illustration, and if the sample is not blood or a test is performed on the sediment in addition to the supernatant, outlets may be provided at lower portions of the first chambers 120.

The outlets of the siphon channels 125-1,125-2 and 125-3 are connected to the second chambers 130-1,130-2 and 130-3. The second chambers 130 may accommodate only blood, or may have a reagent or reactant pre-stored therein to perform pretreatment or first order reaction for blood or to perform a simple test prior to the main test. In the illustrated embodiment, binding between an analyte and a first marker conjugate occurs in at least one of the second chambers 130-1,130-2 and 130-3.

Specifically, the first marker conjugate may stay in at least one of the second chambers 130 in a liquid phase or solid phase. When the marker conjugate is solid phase, the inner wall of the second chambers 130 may be coated with the marker conjugate or the marker conjugate may be temporarily immobilized on a porous pad.

The first marker conjugate may be a complex formed by combining a marker and a capture material which specifically reacts with the analyte. For example, if the analyte is antigen Q, the first marker conjugate may be a conjugate of the marker and antibody Q which specifically reacts with antigen Q.

The marker may be one of latex beads, metal colloids including gold colloids and silver colloids, enzymes including peroxidase, fluorescent materials, luminous materials, superparamagnetic materials, materials containing lanthanum (III) chelates, and radioactive isotopes. However, embodiments of the present invention are not limited thereto.

As an example, NT-proBNP may be used as the analyte, and NT-proBNP antibody may be used as the capture material.

As another example, for hepatitis B diagnosis, HbsAg(Hepatitis B Surface Antigen) may be used as the analyte, and HBsAb(Hepatitis B Surface Antibody) may be used as the capture material. Also, if test paper on which a chromatographic reaction occurs is inserted into the reaction chamber 150, which will be described later, a second marker conjugate which binds with a second capture material immobilized on the control line of the test paper to confirm reliability of the reaction may be included in the second chamber 130. The second marker conjugate may also be in a liquid phase or solid phase and, when in solid phase, the inner wall of the second chamber 130 may be coated with the second marker conjugate or the second marker conjugate may be temporarily immobilized on a porous pad.

The second marker conjugate binding with the second capture material immobilized on the control line is a conjugate of the marker and a material specifically reacting with the second capture material immobilized on the control line. The marker may be one of the aforementioned exemplary materials. If the second capture material immobilized on the control line is biotin, a conjugate of streptavidin and the marker may be temporarily immobilized in the second chamber 130.

Accordingly, when blood flows into the second chamber 130, antigen Q present in the blood binds with the first marker conjugate having antibody Q and is discharged to the third chamber 140,. At this time, the second marker conjugate having streptavidin is also discharged.

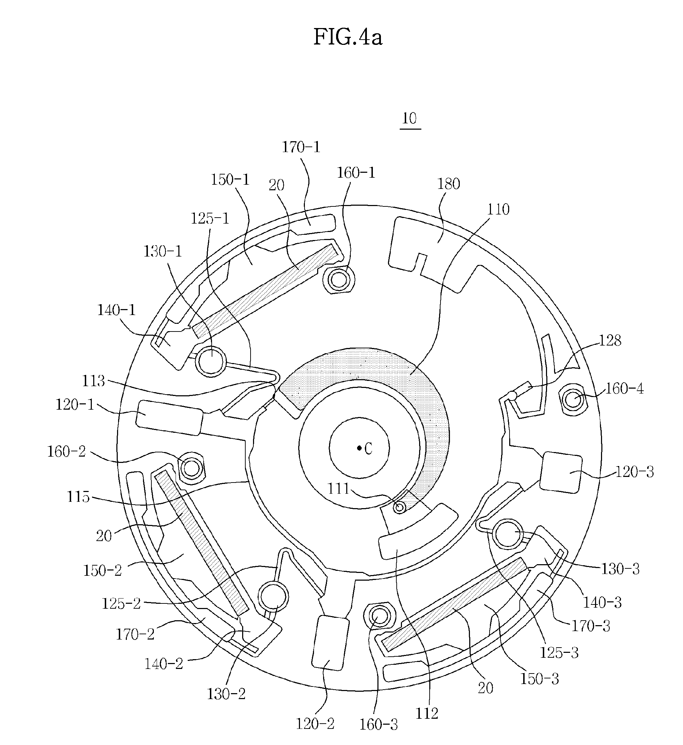

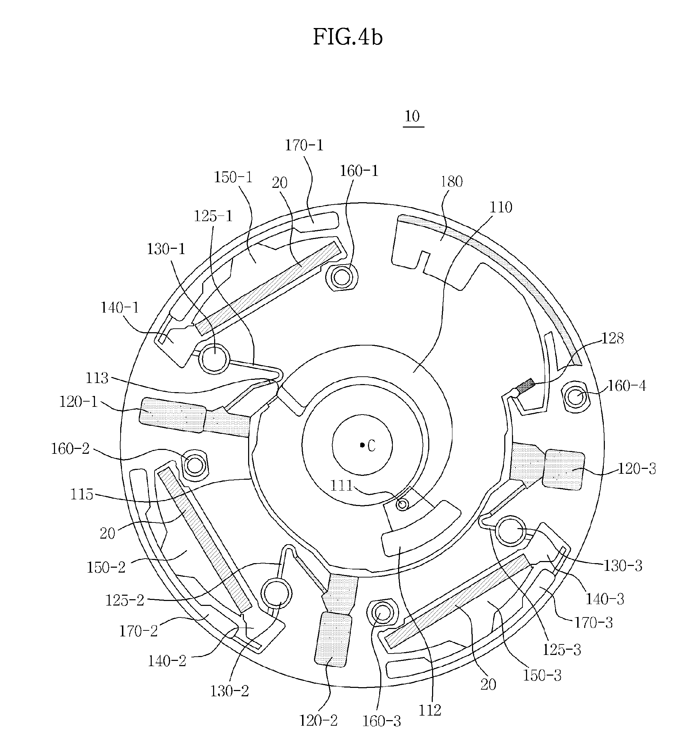

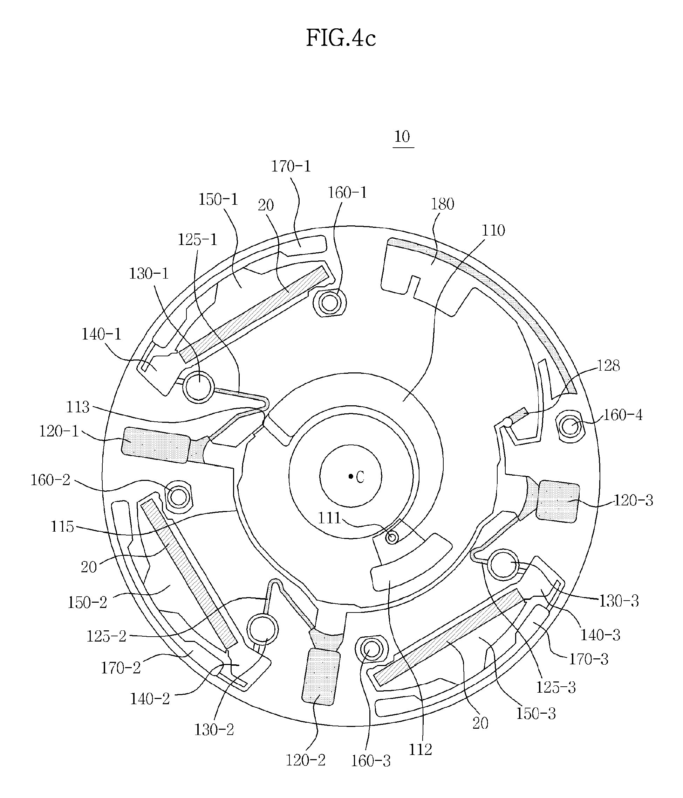

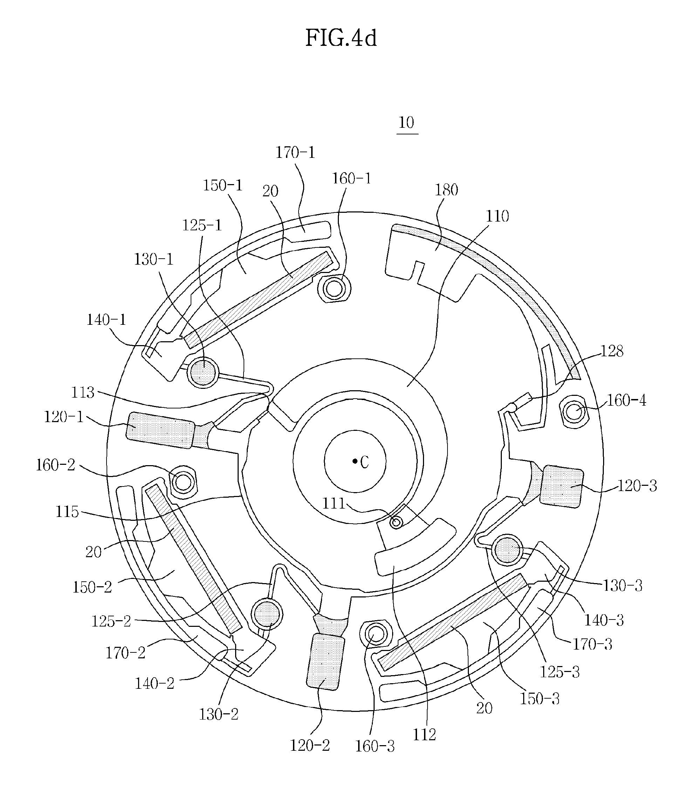

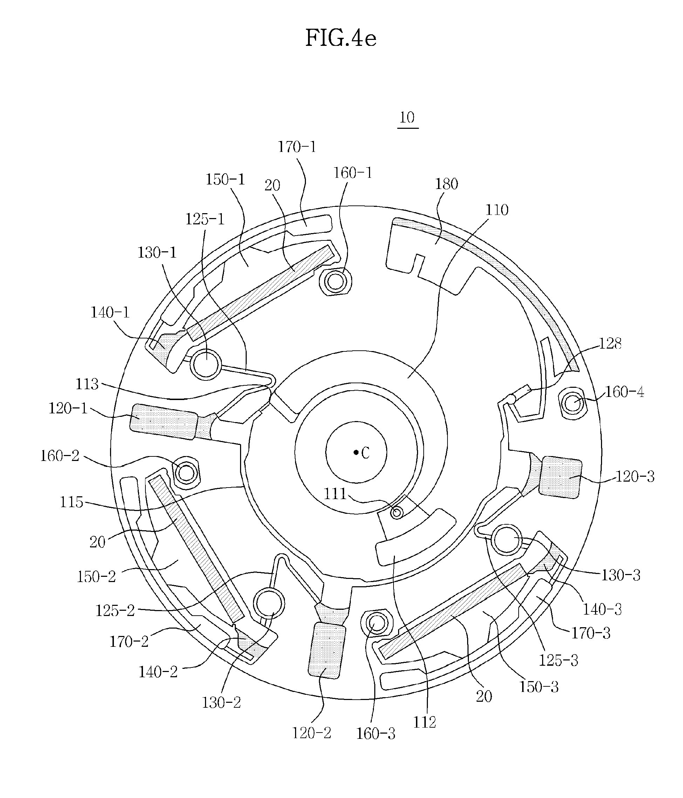

The second chambers 130-1,130-2 and 130-3 are connected to the third chambers 140-1,140-2 and 140-3, and in the illustrated embodiment, the third chambers 140 are used as metering chambers. The metering chambers 140 function to meter a fixed amount of blood accommodated in the second chamber 130 and supply the fixed amount of blood to forth chambers 150. The metering operation of the metering chambers will be described below with reference to FIG. 4.

The residue in the metering chambers 140 which has not been supplied to the fourth chambers 150 is transferred to waste chambers 170.

The third chambers 140-1,140-2 and 140-3 are connected to the reaction chambers 150-1,150-2 and 150-3 which are the fourth chambers. A reaction may occur in the reaction chambers 150 in various ways, and in the illustrated embodiment, chromatography based on capillary pressure is used in at least one of the reaction chambers 150-1,150-2 and 150-3. To this end, the reaction chamber 150 includes a test paper-type detection object 20 to detect presence of an analyte through chromatography.

The reaction chambers 150-1, 150-2 and 150-3 are connected to the fifth chambers, i.e., the waste chambers 170-1, 170-2 and 170-3. The waste chambers 170-1, 170-2 and 170-3 accommodate impurities discharged from the reaction chambers 150-1, 150-2 and 150-3.

In addition to chambers in which a sample or residue is accommodated or a reaction occurs, the platform 100 may be provided with magnetic body accommodating chambers 160-1,160-2,160-3 and 160-4 for position identification. The magnetic body accommodating chambers 160-1,160-2,160-3 and 160-4 accommodate a magnetic material, and the magnetic material accommodated in magnetic body accommodating chambers 160-1, 160-2, 160-3 and 160-4 may be formed of a ferromagnetic material such as iron, cobalt and nickel which have a high intensity of magnetization and form a strong magnet like a permanent magnet, or a paramagnetic material such as chromium, platinum, manganese, and aluminum which have a low intensity of magnetization and thus do not form a magnet when alone but may become a magnet when a magnet approaches thereto to increase the intensity of magnetization thereof.

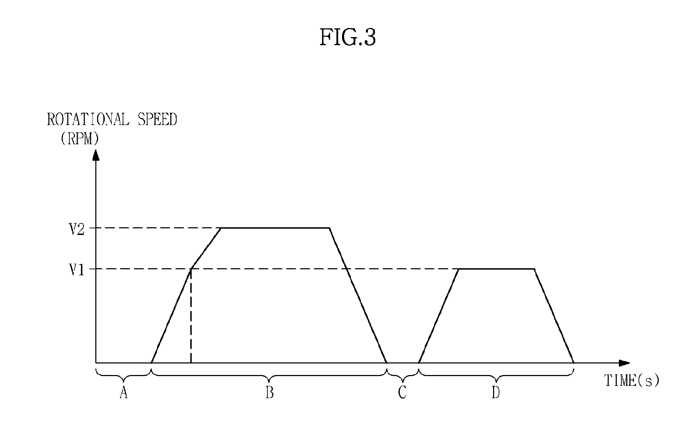

FIG. 3 is a graph schematically illustrating the rotational speed of the platform 100 during respective fluid transfer operations in the microfluidic device according to the exemplary embodiment of the present invention, and FIGS. 4A to 4E are plan views illustrating flow of a fluid in the microfluidic device according to the exemplary embodiment. The microfluidic device of FIGS. 4A to 4E has the same structure as the microfluidic device shown in FIG. 2.FIGS. 2A to 2C.

Referring to FIG. 3, the operation of transferring the fluid in the microfluidic device 10 may be broadly divided into introducing a sample (A), distributing the sample (B), wetting a siphon channel (C), and transferring the sample (D). Here, wetting refers to an operation of the fluid filling the siphon channel 125. Hereinafter, operations of the microfluidic device will be described with reference to the graph of FIG. 3 and the plan views of FIGS. 4A to 4E showing the respective operations.

FIG. 4A is a plan view of the microfluidic device which is in the operation of introducing a sample (A). A sample is introduced into the sample supply chamber 110 through the sample introduction inlet 111 while the platform 100 is at rest (rpm=0). In the present embodiment, a blood sample is introduced. Since a backflow receiving chamber 112 is arranged at a portion adjacent to the sample introduction inlet 111, contamination of the microfluidic device 10 due to blood dropped at a place other than the sample introduction inlet 111 may be prevented during the operation of introducing the sample.

FIG. 4B is a plan view of the microfluidic device which is in the operation of distributing the sample (B). When introduction of the sample is completed, distribution of the sample to the first chambers 120 is initiated. At this time, the platform 100 begins to rotate and the rotational speed (rpm) thereof increases. If a test is performed on a blood sample as in the illustrated embodiment, centrifugation may be performed along with distribution of the sample. Through the centrifugation, the blood may separate into the supernatant and the sediment. The supernatant includes serum and plasma, and the sediment includes corpuscles. The sample used in the test is substantially the supernatant.

As illustrated in FIG. 3, the rotational speed is increased to v1 to distribute the blood accommodated in the sample supply chamber 110 to the "1-1 "th chamber 120-1, the "1-2"th chamber 120-2 and the "1-3"th chamber 120-3 using centrifugal force, and further increased to v2 to allow centrifugation to occur in each chamber. When the blood accommodated in each chamber is centrifuged, the supernatant gathers at a position proximal to the center of rotation, while the sediment gathers at a position distal to the center of rotation. In the embodiment of FIGS. 4A to 4E, the first chambers 120 have the same size. However, the first chambers 120 may respectively have different sizes depending on the amounts of fluid to be distributed thereto.

When supply of blood to the "1-1 "th chamber 120-1, the "1-2"th chamber 120-2 and the "1-3"th chamber 120-3 is completed, a part of the blood not supplied to the first chambers 120 but remaining in the sample supply chamber 110 flows into the QC chamber 128 through the distribution channel 115, and the remaining part thereof which does not flow into the QC chamber 128 flows into the excess chamber 180.

As shown in FIG. 4B, a magnetic body accommodating chamber 160-4 is formed at a position adjacent to the QC chamber 128, and thus a magnet of a detection module, which will be described later, may cause the QC chamber 128 to face a light receiving element of the detection module. Accordingly, when the light receiving element faces the QC chamber 128, it may measure transmittance of the QC chamber 128 and determine whether supply of blood to the first chambers 120 has been completed.

FIG. 4C is a plan view of the microfluidic device which is in the operation of wetting a siphon channel (C). When distribution and centrifugation of the blood are completed, the platform 100 is stopped (rpm=0), and when the platform 100 is stopped, the blood accommodated in the first chambers 120-1, 120-2 and 120-3 is caused to fill the siphon channels 125-1,125-2 and 125-3 by the capillary pressure.

FIG. 4D is a plan view of the microfluidic device which is in the operation of transferring the sample to the second chamber 130 during the operation of transferring the sample (D). When wetting of the siphon channel 125 is completed, the platform 100 is rotated again to allow the blood filling the siphon channels 125-1,125-2 and 125-3 to flow into the second chambers 130-1,130-2 and 130-3. As shown in FIG. 4D, since the inlets of the siphon channels 125-1,125-2 and 125-3 are connected to the upper portions of the first chambers 120-1, 120-2 and 120-3 (the portions proximal to the center of rotation), the supernatant flows into the second chambers 130-1,130-2 and 130-3 through the siphon channels 125-1,125-2 and 125-3.

The second chambers 130 may simply serve to temporarily accommodate the blood flowing thereinto, or allow, as described above, binding between a specific antigen in the blood and the marker conjugate 22a' from the marker conjugates 22a' and 23a pre-provided in the second chambers 130 to occur.

FIG. 4E is a plan view of the microfluidic device which is in the operation of transferring the sample to the metering chambers 140 during the operation of transferring the sample (D). The blood flowing into the second chambers 130-1,130-2 and 130-3 are then introduced into the third chambers, i.e., the metering chambers 140-1,140-2 and 140-3 by centrifugal force. By centrifugal force, the metering chambers 140-1,140-2 and 140-3 are filled with blood from the lower portion thereof, i.e., from the portion distal to the center of rotation. After the metering chambers 140-1,140-2 and 140-3 is filled with blood up to the outlets thereof, which are connected to the fourth chambers, i.e., the reaction chambers 150-1,150-2 and 150-3, blood subsequently introduced into the metering chambers 140-1,140-2 and 140-3 flows into the reaction chambers 150-1,150-2 and 150-3 through the outlets of the metering chambers 140-1,140-2 and 140-3. Therefore, the positions of the outlets of the metering chambers 140 may be adjusted to supply a fixed amount of blood to the reaction chambers 150.

The reaction occurring in the reaction chambers 150 may be immunochromatography or a binding reaction with a capture antigen or capture antibody, as described above.

As shown in FIG. 4E, if the magnetic body accommodating chambers 160-1, 160-2 and 160-3 are formed at positions adjacent to the reaction chambers 150-1,150-2 and 150-3, the positions of the reaction chambers 150-1,150-2 and 150-3 may be identified by a magnet of the detection module, which will be described later.

### <Test Device-Seating the Microfluidic Device>

Hereinafter, a detailed description will be given of a test device according to one embodiment of the present invention.

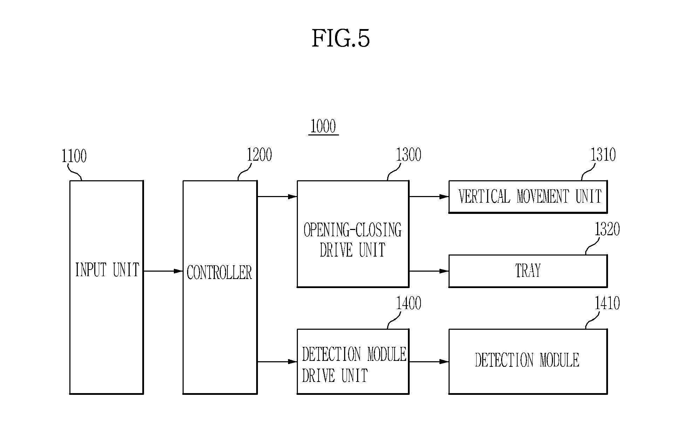

Constituents related to seating and rotating the microfluidic device will be described with reference to FIGS. 5 to 14.

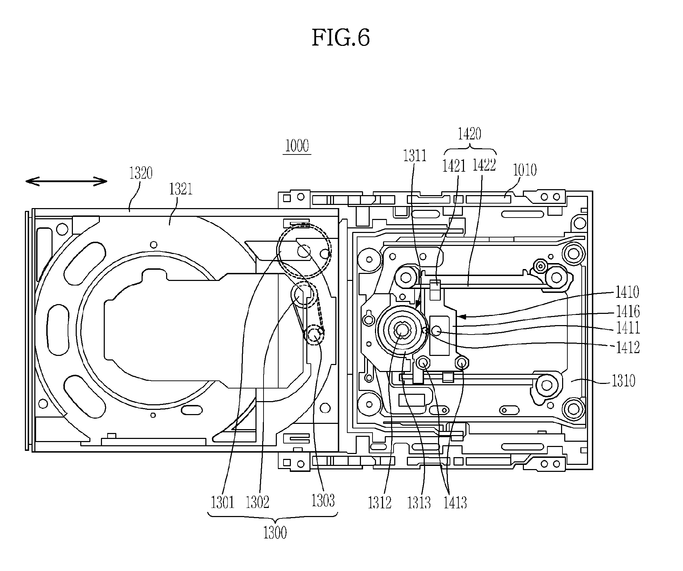

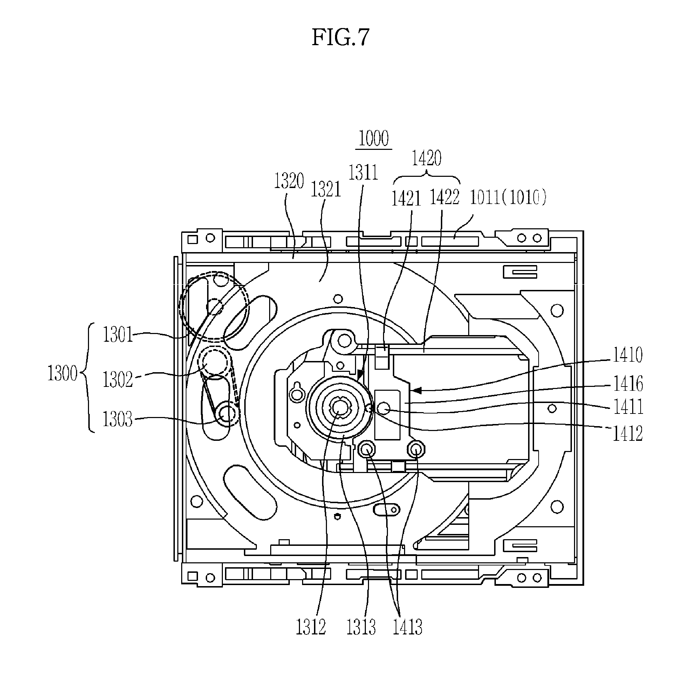

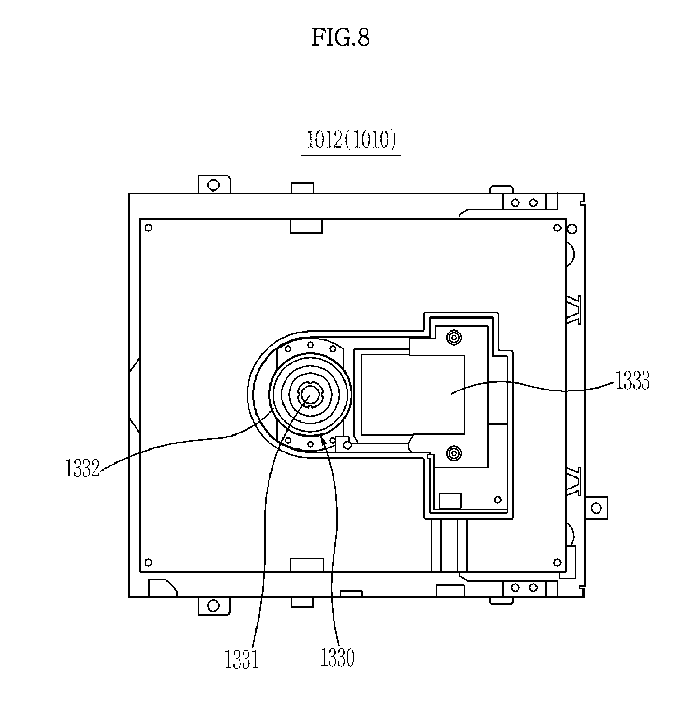

FIG. 5 is a function block diagram of the test device according to one exemplary embodiment, FIG. 6 is a plan view illustrating a tray according to the exemplary embodiment of the present invention, which is in an open state, and FIG. 7 is a plan view illustrating the tray according to the exemplary embodiment, which is in a closed state, and FIG. 8 is a plan view illustrating the bottom surface of an upper housing according to the exemplary embodiment of the present invention.

As shown in FIGS. 5 to 8, a test device 1000 includes an input unit 1100 allowing a user to input a command therethrough from the outside, a controller 1200 to control overall operation and functions of the test device 1000 according to a command from the input unit 1100, an opening-closing drive unit 1300 to drive a vertical movement unit 1310 or a tray 1320 according to a command from the controller 1200, and a detection module drive unit 1400 to drive a detection module 1410 according to a command from the controller 1200.

The controller 1200 controls operations related to the rotary drive unit 1340 of the test device 1000, and controls operations of the tray 1320 and the vertical movement unit 1310 to mount the microfluidic device 10 on the test device 1000. Also, the controller 1200 controls operation of the detection module 1410 to perform a reaction related to detection of the detection object 20 in the microfluidic device 10.

The rotary drive unit 1340 rotates the microfluidic device 10 using a spindle motor. The rotary drive unit 1340 may receive a signal output from the controller 1200 to repeat the operation of rotating and stopping, thereby generating centrifugal force to transfer the fluid in the microfluidic device 10 or moving various structures in the microfluidic device 10 to desired positions. Also, the rotary drive unit 1340 may include a motor drive to control the angular position of the microfluidic device 10. For example, the motor drive may employ a stepper motor or a direct current (DC) motor.

The microfluidic device 10 is seated on the tray 1320. The tray 1320 may be moved to a first state in which the tray 1320 is positioned outside the test device 1000 (FIG. 6) and to a second state in which the tray 1320 is positioned inside the test device 1000 (FIG. 7).

The vertical movement unit 1310 may perform a first movement during which the vertical movement unit 1310 moves to an upper side at which the tray 1320 is arranged and a second movement which is performed in the direction opposite to the first movement. Through movements of the vertical movement unit 1310, the microfluidic device 10 may be correctly seated on the tray 1320. Also, by force acting between the clamper 1330 positioned at the housing 1010 and the rotary drive unit 1340, the clamper 1330 may press the microfluidic device 10. For example, the force acing between the clamper 1330 and the rotary drive unit 1340 may be magnetic force. Accordingly, the microfluidic device 10 may be seated at a set position on the test device 1000, and thereby errors in a test such as failure to perform the test due to failure to seat the microfluidic device 10 at the set position may be prevented. Also, since the microfluidic device 10 is pressed by the clamper 1330, displacement of the microfluidic device 10 from the tray 1320 may be prevented during operation of the test device 1000.

The microfluidic device 10 may be loaded onto the tray 1320, and microfluidic structures and a magnetic body 161 may be positioned in the microfluidic device 10. The detection object 20 is positioned in at least one of the microfluidic structures. For the magnetic body 161, a ferromagnetic material or paramagnetic material may be used. The detection object 20 may be an assay site or test paper. The platform 100 of the microfluidic device 10 may be a non-optical bio disc or optical bio disc.

The detection module 1410 may include a light receiving element 1411. Also, the detection module 1410 may include at least one magnet 1413 to guide the microfluidic device 10 to the exact position thereof. In addition, the detection module 1410 may move in the housing 1010 of the test device 1000. For the detection module 1410, the movement of the detection module 1410 performed in the direction in which the tray 1320 is accommodated in the housing 1010 is defined as a third movement, and the movement thereof performed in the direction opposite to the third movement is defined as a fourth movement. That is, when the microfluidic device 10 is seated on the tray 1320, the third movement and the fourth movement are performed in a radial direction of the microfluidic device 10. Thereby, through the movements of the detection module 1410, the light receiving element 1411 may be moved to perform detection at various positions and further the microfluidic device 10 may be seated at a set position by attractive force created between the magnet 1413 in the detection module 1410 and the magnetic body 161 in the microfluidic device 10.

The test device 1000 includes the housing 1010 forming an external appearance thereof, the tray 1320 onto which the microfluidic device 10 is loaded, and the vertical movement unit 1310 positioned inside the housing 1010 and coupled to the microfluidic device 10 to rotate the microfluidic device 10.

The housing 1010 forming the external appearance may include an upper housing 1012 and a lower housing 1011 In one embodiment, a light emitting element 1333 may be positioned at the upper housing 1012, and a vertical movement unit 1310 may be positioned at the lower housing 1011. The vertical movement unit 1310 may be arranged at the lower side of the tray 1320. The tray 1320 may be accommodated in the lower housing in the second state. The detection module 1410 may be arranged at the vertical movement unit 1310. As the detection module 1410 is provided with the light receiving element 1411, the light emitting element 1333 and the light receiving element 1411 may be arranged such that the microfluidic device 10 is placed therebetween.

The tray 1320 may be provided with a seating surface 1321 on which the microfluidic device 10 is seated. The seating surface 1321 is formed in a shape corresponding to that of the microfluidic device 10 to stably support the microfluidic device 10. The tray 1320 may be moved to the first state and the second state by an opening-closing drive unit 1300 positioned at the lower side of the tray 1320. The tray 1320 is open in the first state, and is closed in the second state. The tray 1320 may be controlled by the controller 1200 to be opened or closed. Alternatively, the tray 1320 may be opened and closed by control of a dedicated OPEN/CLOSE button instead of the controller 1200.

The vertical movement unit 1200 may be provided with a rotary drive unit 1340 which is inserted into a through hole of the microfluidic device 10 through an opening of the tray 1320. The rotary drive unit 1340 is coupled to the microfluidic device 10 to rotate the microfluidic device 10. A head portion 1311 coupled to the microfluidic device 10 is provided at the upper side of the rotary drive unit 1340. The head portion 1311 may be provided with an insert portion 1312 inserted into the through hole of the microfluidic device 10, and a support portion 1313 adapted to contact the lower surface of the microfluidic device 10 when the rotary drive unit 1340 is completely inserted into the through hole. With the microfluidic device 10 mounted on the rotary unit 1340, the rotary drive unit 1340, performs the first movement toward the position of the tray 1320, and is thus couped in the through hole of the microfluidic device 10. After the rotary drive unit 1340 is coupled in the through hole, it rotates the microfluidic device 10.

The vertical movement unit 1310 may be caused to perform the first movement of moving toward the position of the tray 1320 by the opening-closing drive unit 1300, and the second movement which is made in the direction opposite to the first movement. According to the movement of the vertical movement unit 1310, the rotary drive unit 1340 is also moved. The vertical movement unit 1310 may be moved by the opening-closing drive unit 1300 located at the lower side of the tray 1320.

The opening-closing drive unit 1300 includes a drive motor 1301. The drive motor 1301 is coupled to a gear (not shown), which is in turn coupled to a drive pulley 1302. The drive pulley 1302 transmits power to a driven pulley 1303. The driven pulley 1303 is coupled to the tray 1320 such that the tray 1320 is moved to the first state and the second state. According to the illustrated embodiment, when the tray 1320 is moved to the second state, the vertical movement unit 1310 automatically performs the first movement toward the tray 1320.

Mounted at one side of the vertical movement unit 1310 is the detection module 1410. The detection module 1410 may include a component to detect the detection object 20 of the microfluidic device 10. The detection module 1410 may include a plate 1416 at which the component is positioned. The detection module 1410 may be slidably moved by a guide member 1420 provided at the vertical movement unit 1310. The guide member 1420 includes a support rod 1422 and a coupling portion 1421 protruding to the upper side of the plate 1416. The plate 1416 may be coupled to the support rods 1422 to move within the housing 1010 along the support rods 1422, from an inner circumference to an outer circumference. The coupling portion 1421 is slidably mounted to the support rod 1422 to support the detection module 1410 and at the same time to allow the detection module 1410 to move along the support rods 1422.

The upper housing 1010 is positioned at the upper side of the tray 1320. The upper housing 1010 is provided with the clamper 1330 to fix the microfluidic device 10 to the rotary drive unit 1340. The clamper 1330 may include the accommodation portion 1331 to accommodate the rotary drive unit 1340 and the protrusion 1332 protruding to form a protruding outer circumference to be firmly coupled to the rotary drive unit 1340. The clamper 1330 may be arranged to execute movement with respect to the upper housing 1010.

The accommodation portion 1331 of the clamper 1330 may be provided with a magnetic body, and a magnet may be provided at the upper portion of the rotary drive unit 1340. Alternatively, the accommodation portion 1331 of the clamper 1330 may be provided with a magnet, and a magnetic body may be provided at the upper portion of the rotary drive unit 1340.

When the through hole of the microfluidic device 10 loaded onto the tray 1320 is seated on the rotary drive unit 1340, the clamper 1330 is allowed to press the microfluidic device 10 using the magnetic force generated between the clamper 1330 and the rotary drive unit 1340. Thereby, shaking of the microfluidic device 10 may be prevented during operation thereof, and the through hole of the microfluidic device 10 may remain seated on the rotary drive unit 1340 even when attractive force acts between the magnet 1413 mounted to the detection module 1410 and the magnetic body 161 of the microfluidic device 10. To this end, the magnetic force between the clamper 1330 and the rotary drive unit 1340 is set to be stronger than the magnetic force between the magnetic body 161 mounted to the microfluidic device 10 and the magnet 1413 mounted to the detection module 1410.

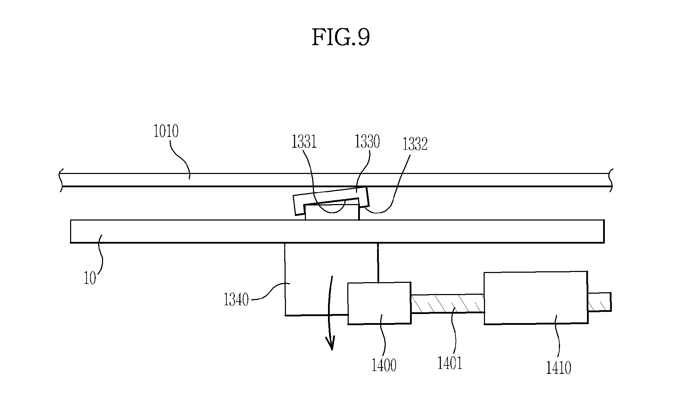

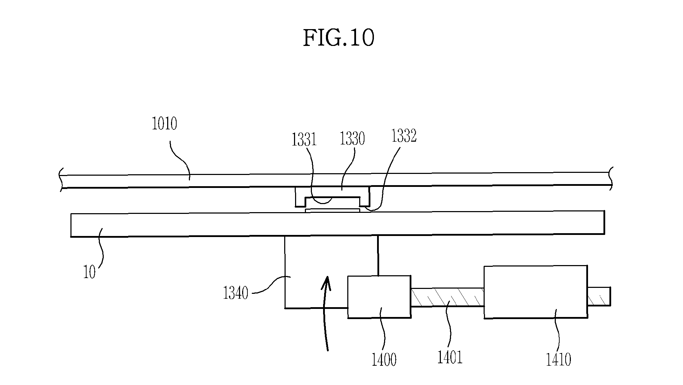

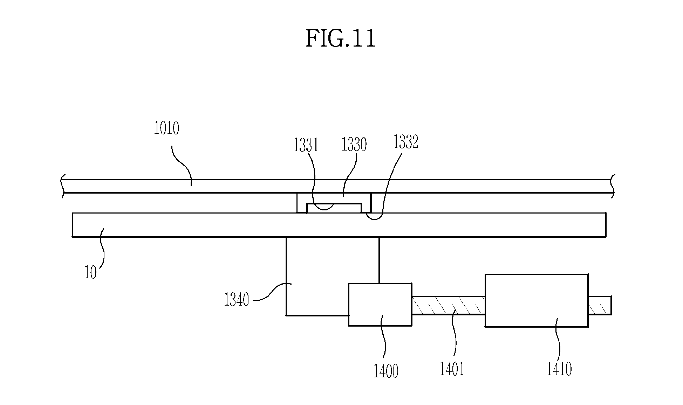

FIG. 9 is a view illustrating the microfluidic device according to one embodiment of the present invention, which is abnormally coupled to a rotary drive unit and a clamper, FIG. 10 is a view illustrating the microfluidic device according to the embodiment of the present invention, which is separated from the rotary drive unit and the clamper, and FIG. 11 is a view illustrating the microfluidic device according to the embodiment of the present invention, which is normally coupled to a rotary drive unit and a clamper.

As shown in FIGS. 9 to 11, the rotary drive unit 1340 according to the illustrated embodiment of the present invention is vertically movable. Independently of the rotary drive unit 1340, the detection module 1410 is movable within the housing 1010. The rotary drive unit 1340 is moved by the opening-closing drive unit 1300, while the detection module 1410 is moved by the detection module drive unit 1400.

The through hole of the microfluidic device 10 is seated on the rotary drive unit 1340. The rotary drive unit 1340 may perform the first movement and the second movement at least once. Accordingly, if the rotary drive unit 1340 is abnormally coupled to the clamper 1330 as shown in FIG. 9, the rotary drive unit 1340 may perform the second movement. By the second movement of the rotary drive unit 1340, the rotary drive unit 1340 and the clamper 1330 are arranged spaced apart from each other as shown in FIG. 10. Since separation of the microfluidic device 10 from the clamper 1330 causes the tray 1320 to be opened, the rotary drive unit 1340 performs the second movement only to an extent to which the rotary drive unit 1340 is not spaced apart from the microfluidic device 10. When the rotary drive unit 1340 performs the first movement after the second movement, the rotary drive unit 1340 is coupled to the clamper 1330 through the microfluidic device 10 and the tray 1320, as shown in FIG. 11.

The rotary drive unit 1340 may be moved by movement of the vertical movement unit 1310. For the detection module 1410, power produced by the detection module drive unit 1400 is transmitted to the detection module 1410 through the power transmission part 1401 to allow the detection module 1410 to move in a radial direction. Thereby, if the rotary drive unit 1340 is abnormally coupled to the clamper 1330, errors may be corrected without user intervention.

In conventional cases, if the clamper 1330 arranged at the upper housing 1010 is abnormally seated between the microfluidic device 10 and the upper housing 1010, the microfluidic device 10 fails to rotate. In the illustrated embodiment, however, a gap is created between the rotary drive unit 1340 and the clamper 1330 by the second movement of the rotary drive unit 1340, and therefore even when the clamper 1330 is abnormally seated, an error is corrected by allowing the clamper 1330 to be normally seated.

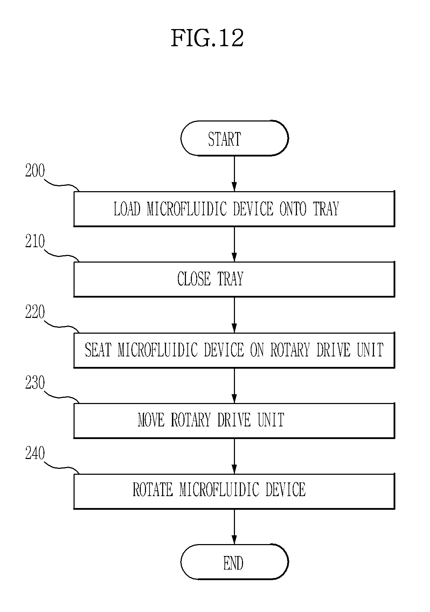

FIG. 12 is a flowchart illustrating a control method of a test device according to an exemplary embodiment.

As shown in FIG. 12, a control method of the test device 1000 includes loading the microfluidic device 10 onto the tray 1320 (200), seating the center of the microfluidic device 10 on the rotary drive unit 1340 (220), and performing, by the rotary drive unit 1340, a vertical movement at least once (230).

First, the microfluidic device 10 is loaded onto the tray 1320 (200). The tray 1320 is moved to the second state so as to be accommodated in the housing 1010 and closed (210). When the tray 1320 is closed, the rotary drive unit 1340 is coupled to the through hole of the microfluidic device 10 and the microfluidic device 10 is seated on the rotary drive unit 1340 (220). The rotary drive unit 1340 may move to be normally coupled to the clamper 1330 such that the microfluidic device 10 is coupled to the clamper 1330 (230). According to the illustrated embodiment, even when the first movement of the rotary drive unit 1340 is performed once and allows the rotary drive unit 1340 to be coupled to the microfluidic device 10, the rotary drive unit 1340 may make the second movement and then the first movement. As such, the rotary drive unit 1340 may perform the first movement and the second movement at least once. Thereby, when the clamper 1330 is incorrectly coupled to the rotary drive unit 1340 through the first movement of the rotary drive unit 1340, the position of the clamper 1330 may be corrected by performing the second movement of the rotary drive unit 1340 and then the first movement again. In case that the rotary drive unit 1340 is coupled to the vertical movement unit 1310 as in the illustrated embodiment, the vertical movement unit 1310 may perform the first movement and the second movement to move the rotary drive unit 1340.

The controller 1200 controls the second movement made by the rotary drive unit 1340 or vertical movement unit 1310 such that the microfluidic device 10 is not separated from the rotary drive unit 1340 during movement. This is because the second movement of the rotary drive unit 1340 beyond a certain distance could cause the tray 1320 to be opened, as the vertical movement unit 1310 and the tray 1320 are moved by the opening-closing drive unit 1300. Since the rotary drive unit 1340 needs to be moved by a distance that does not cause the tray 1320 to be opened, the rotary drive unit 1340 is moved to an extent that does not cause the microfluidic device 10 to be displaced from the rotary drive unit 1340. When the microfluidic device 10 is normally seated on the rotary drive unit 1340 through the above operations, the microfluidic device 10 begins to rotate (240).

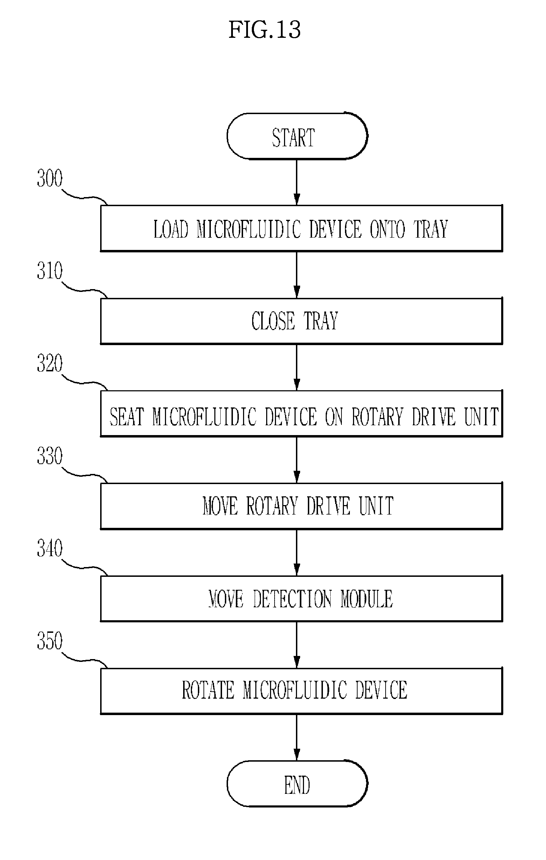

FIG. 13 is a flowchart illustrating a control method of a test device according to another embodiment of the present invention.

According to the illustrated embodiment shown in FIG. 13, in addition to movement of the rotary drive unit 1340 (330), the detection module 1410 may be moved (340)._Compared to the rotary drive unit 1340, the detection module 1410 may perform a third movement in which the detection module 1410 moves to one side of the housing 1010, and a fourth movement which is performed in the direction opposite to that of the third movement. Due to provision of at least one magnet 1413 of the detection module 1410 and the magnetic body 161 of the microfluidic device 10, the detection module 1410 is moved to cause the microfluidic device 10 to be stably seated. Through movement of the detection module 1410, fine adjustment of the position of the microfluidic device 10 is allowed when the microfluidic device 10 is seated.

When the microfluidic device 10 is coupled to the clamper 1330, the detection module 1410 may be moved to the outermost circumference of the housing 1010. This serves to prevent the magnet 1413 from affecting rotation of the microfluidic device 10 during assay using the microfluidic device 10.

In conventional cases, movement of the detection module 1410 has caused the microfluidic device 10 to move and fail to normally rotate. However, in the illustrated embodiment, seating of the microfluidic device 10 is controlled not only by the movement of the detection module 1410 but also by the movement of the rotary drive unit 1340, and therefore movement of the detection module 1410 may be prevented from causing operational errors.

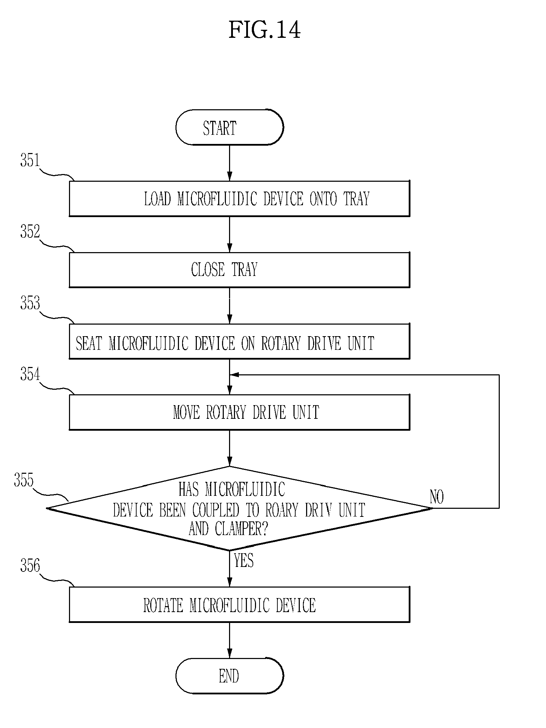

FIG. 14 is a flowchart showing a control method of a test device according to another embodiment of the present invention.

In accordance with the illustrated embodiment shown in FIG. 14, after the rotary drive unit 1340 has moved, the controller 1200 may check if the microfluidic device 10 is properly coupled to the rotary drive unit 1340 and the clamper 1330 (355). For example, the coupling may be checked through the conditions of the rotary drive unit 1340 and the clamper 1330. If the rotary drive unit 1340 and the clamper 1330 are properly coupled, magnetic force is produced between the clamper 1330 and the rotary drive unit 1340. Based on this magnetic force, normal seating of the microfluidic device 10 on the rotary drive unit 1340 may be verified. Thereby, if the microfluidic device 10 is normally seated, the microfluidic device 10 begins to rotate (356). If the microfluidic device 10 is not normally seated, the first movement and the second movement of the rotary drive unit 1340 are performed again (354).

### <Test Device-Detecting the Detection Object on Various Radii >

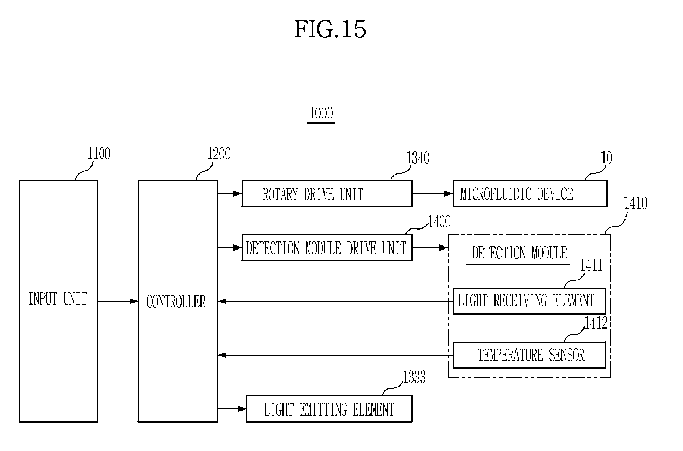

Hereinafter, a detailed description will be given of the test device according to one embodiment of the present invention with reference to FIGS. 15 to 28.

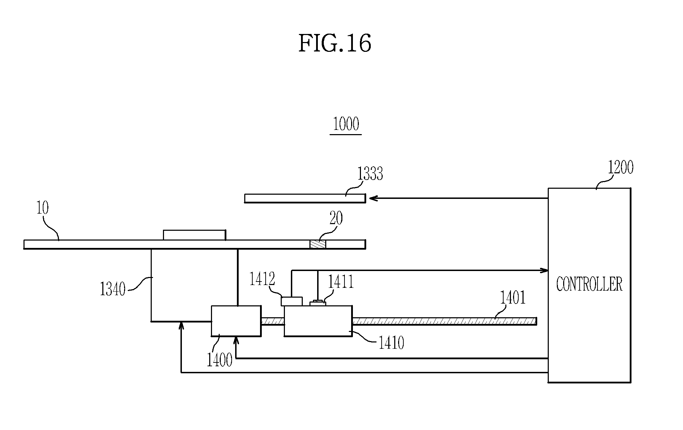

FIG. 15 is a block diagram showing the configuration of a test device according to one embodiment of the present invention and FIG. 16 is a conceptual side view illustrating the configuration of the test device according to the embodiment of the present invention.

According to the illustrated embodiment, the test device 1000 includes a rotary drive unit 1340 to rotate the microfluidic device 10, a light emitting element 1333 to emit light to the microfluidic device 10, a detection module 1410 provided with a light receiving element 1411 to detect the detection object 20 through the light emitted from the light emitting element 1333 and a temperature sensor 1412 to sense the temperature of the detection object 20, a detection module drive unit 1400 to move the detection module 1410 in a radial direction, an input unit 1100 allowing a user to input a command therethrough from the outside, and a controller 1200 to control overall operations and functions of the test device 1000 according to the command input through the input unit 1100.

The microfluidic device 10 of the present embodiment is the same as the microfluidic device 10 described above with reference to FIGS. 1 to 4, and thus a detailed description thereof will be omitted.

When the microfluidic device 10 is loaded, the rotary drive unit 1340, which may be realized with a spindle motor, is controlled by the controller 1200 to rotate the microfluidic device 10. The rotary drive unit 1340 may receive a signal output from the controller 1200 and repeat the operation of rotation and stop, thereby generating centrifugal force to transfer the fluid of the microfluidic device 10 or moving various structures on the microfluidic device 10 to desired positions.

Also, the rotary drive unit 1340 may include a motor drive to control the angular position of the microfluidic device 10. For example, the motor drive may employ a stepper motor or a DC motor.

The light emitting element 1333 may be a surface light source having a large light emitting area to uniformly emit light on a certain region of the microfluidic device 10. For example, a back light unit may be used as the light emitting element 1333.

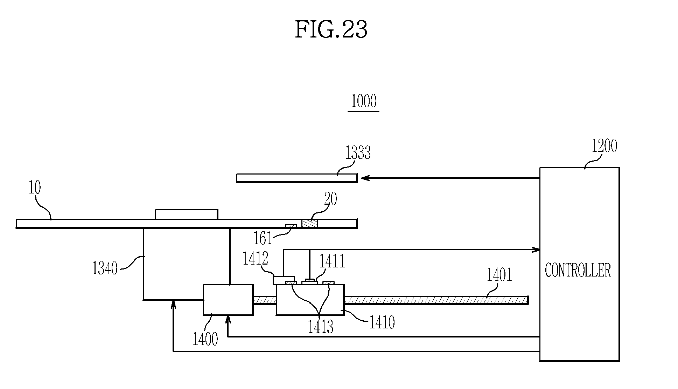

The light emitting element 1333 may be arranged to face in the same direction as the light receiving element 1411, or it may be arranged to face the light receiving element 1411 as shown in FIG. 23. FIG. 23 shows that the light emitting element 1333 is positioned at the upper side of the microfluidic device 10 and the light receiving element 1411 is positioned at the lower side of the microfluidic device 10 such that the microfluidic device 10 is placed between the light emitting element 1333 and the light receiving element 1411. However, the positions of the light emitting element 1333 and the light receiving element 1411 may be changed. The light emitting element 1333 may be controlled by the controller 1200 to adjust the amount of light emitted therefrom.

The light receiving element 1411 receives light reflected from or transmitted through the detection object 20 after being emitted from the light emitting element 1333, and detects the detection object 20. The light receiving element 1411 may be a CMOS image sensor or CCD image sensor.

When the light receiving element 1411 receives light reflected from or transmitted through the detection object 20 and obtains an image of the detection object 20, the controller 1200 detects the presence of the detection object 20, e.g., the analyte in the test paper and the concentration of the analyte, using the image.

According to the illustrated embodiment, the test device 1000 has one light receiving element 1411 installed at the detection module 1410, which is a mechanism movable in a radial direction, such that the light receiving element 1411 may detect a plurality of detection objects 20 provided in the microfluidic device 10.

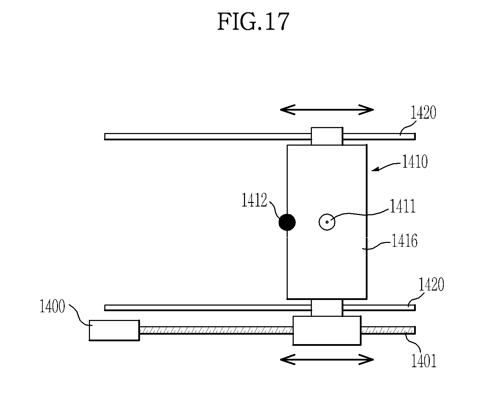

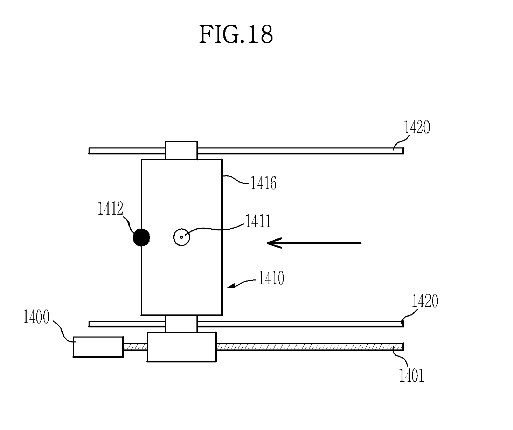

FIGS. 17 and 18 are views illustrating a radial movement of a detection module 1410 according to one embodiment of the present invention, as seen from the top.

Referring to FIGS. 17 and 18, the detection module 1410 may be moved in a radial direction by drive force supplied from the detection module drive unit 140. The detection module drive unit 1400 may be a feeder motor or stepper motor.

The travel distance of the detection module 1410 may be longer than the radius of the microfluidic device 10. The travel distance is sufficient if the detection module 1410 is moved from the outside of the outer circumference of the microfluidic device 10 to a position near the center of the microfluidic device 10.

The detection module 1410 may include a plate 1416 on which a constituent such as the light receiving element 1411 or temperature sensor 1412 is installed. The detection module 1410 may be slidably moved by two guide members 1420 which guide stable radial movement. The guide members 1420 may be formed in a rod shape, and the plate 1416 may be coupled to the guide members 1420 to move along the guide members 1420. The plate 1416 is slidably mounted on the guide members 1420 to support the detection module 1410 and allow the detection module 1410 to move along the guide members 1420.

Also, the detection module 1410 is mounted on a power transmission part 1401 such that power produced by the detection module drive unit 1400 is transmitted to the detection module 1410 through the power transmission part 1401 to move the detection module 1410 in a radial direction. That is, when the detection module drive unit 1400 is actuated and the power thereof is transmitted to the detection module 1410 through the power transmission part 1401, the detection module 1410 moves along the power transmission part 1401 and the guide member 1420 in a radial direction.

FIG. 16 shows that the detection module 1410 is positioned under the microfluidic device 10 and the light emitting element 1333 is positioned over the microfluidic device 10. However, this configuration is simply an example and the positions of the detection module 1410 and the light emitting element 1333 may be changed.

A temperature sensor 1412 is mounted on the detection module 1410 to detect the temperatures of the detection objects 20 arranged on different radii.

For the temperature sensor 1412, a non-contact temperature sensor such as an infrared temperature sensor, or a contact temperature sensor such as a thermistor, a resistance temperature detector and a thermocouple may be used. In case that the temperature of detection object 20 needs to be kept constant, a non-contact temperature sensor may be used to measure the temperature of the detection object 20 in a non-contact manner.

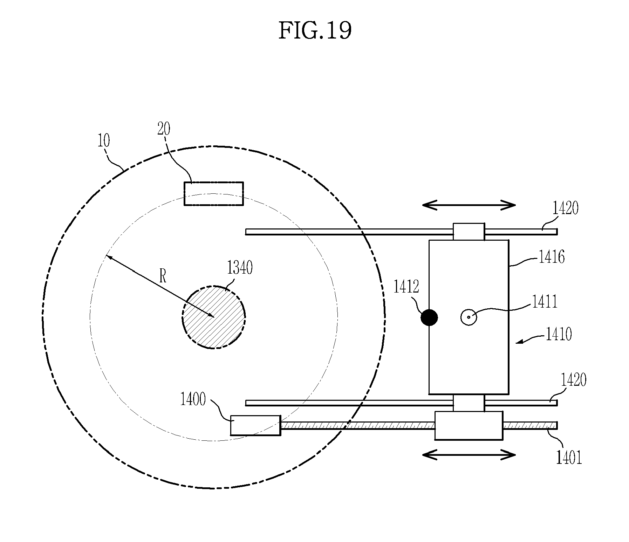

FIGS. 19 to 21 are views illustrating movement of the detection object 20 to a position facing the light receiving element 1411 of the detection module 1410 by movement of the detection module 1410 and rotation of the microfluidic device 10 in the test device 1000 according to one embodiment of the present invention, as viewed from the top.

The microfluidic device 10, which is positioned at the upper side of the detection module 1410, is shown in a dotted line to more clearly show movement of the detection module 1410, which is positioned at the lower side of the microfluidic device 10 and shown in a solid line.

FIG. 19 shows the microfluidic device 10 which has stopped rotating as the reaction in the microfluidic device 10 has been completed. In FIG. 19, the detection module 1410 is positioned below the microfluidic device 10, beyond the outer circumference of the microfluidic device 10. For convenience of description, other structures in the microfluidic device 10 are not shown, and only one detection object 20 is shown.

Here, the detection object 20 may be test paper included in the reaction chamber 150 of the microfluidic device 10 described above to detect presence of the analyte through chromatography.

When the reaction in the microfluidic device 10 is completed and rotation of the microfluidic device 10 is stopped, the controller 1200 controls the detection module drive unit 1400 to move the detection module 1410 in a radial direction such that the detection module 1410 is positioned at a radius R at which the detection object 20 to be detected is positioned.

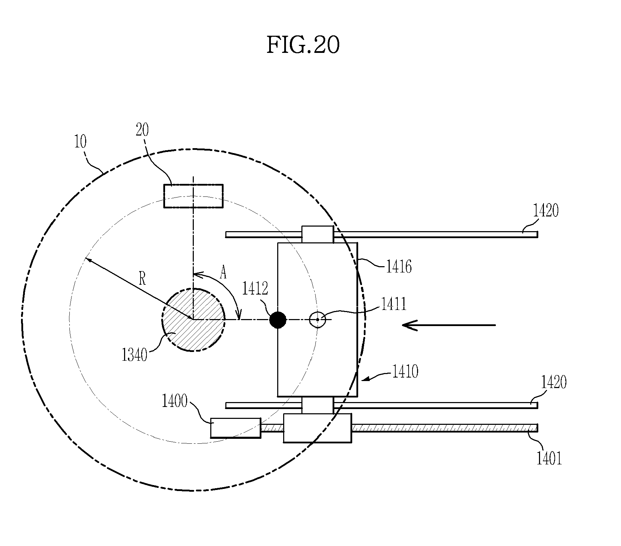

FIG. 20 illustrates that the light receiving element 1411 of the detection module 1410 has reached the radius R at which the detection object 20 of the microfluidic device 10 is installed according to movement of the detection module 1410 toward the center of the microfluidic device 10 in a radial direction.

Information on radii at which the detection objects 20 provided in the microfluidic device 10 are installed may be pre-stored in the controller 1200. When actuating the detection module drive unit 1400, the controller 1200 controls the detection module drive unit 1400 using the information such that the light receiving element 1411 of the detection module 1410 is moved to radius R at which a detection object 20 to be detected is installed.

When the light receiving element 1411 of the detection module 1410 is positioned at radius R at which the detection object 20 of the microfluidic device 10 is positioned, the controller 1200 controls the rotary drive unit 1340 to rotate the microfluidic device 10 such that the detection object 20 of the microfluidic device 10 is moved to a position facing the light receiving element 1411 of the detection module 1410.

The controller 1200 calculates an angle A between the detection object 20 of the microfluidic device 10 and the light receiving element 1411 of the detection module 1410 and controls the rotary drive unit 1340 to rotate the microfluidic device 10 by the angle A.

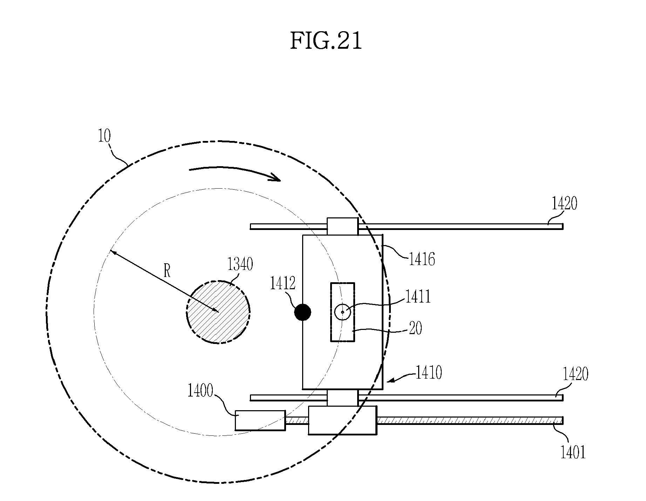

FIG. 21 shows the microfluidic device 10 that has stopped rotating after the detection object 20 of the microfluidic device 10 has moved to the position facing the light receiving element 1411 of the detection module 1410.

When the detection object 20 of the microfluidic device 10 moves to the position facing the light receiving element 1411 of the detection module 1410 and the microfluidic device 10 stops, the light emitting element 1333 is controlled by the controller 1200 to emit light to the detection object 20, and the light receiving element 1411 creates an image of the detection object 20 using the light emitted from the light emitting element 1333 and transmitted through the detection object 20.

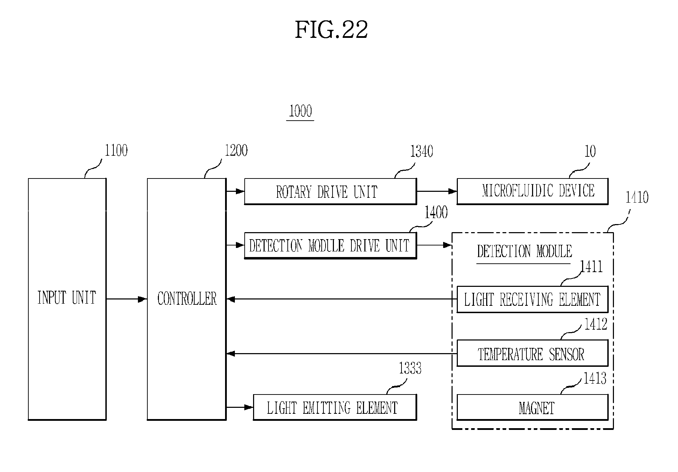

The controller 1200 computes the presence or concentration of the analyte by analyzing the created image of the detection object 20. FIG. 22 is a block diagram illustrating a configuration of a test device 1000 according to another embodiment of the present invention, and FIG. 23 is a conceptual side view illustrating the configuration of the test device 1000 of FIG. 22.

Referring to FIG. 22, the test device 1000 includes a rotary drive unit 1340 to rotate the microfluidic device 10, a light emitting element 1333 to emit light to the microfluidic device 10, a detection module 1410 provided with a light receiving element 1411 to detect the detection object 20 through the light emitted from the light emitting element 1333, at least one magnet 1413 to apply attractive force to the magnetic body 161 of the microfluidic device 10 and a temperature sensor 1412 to sense the temperature of the detection object 20, a detection module drive unit 1400 to move the detection module 1410 in a radial direction, an input unit 1100 allowing a user to input a command therethrough, a controller 1200 to control overall operations and functions of the test device 1000 according to the command input through the input unit 1100.

In the illustrated embodiment, all details other than inclusion of the magnet 1413 in the detection module 1410 are the same as those in the previous embodiment illustrated in FIGS. 15 to 21, and thus a description thereof will be omitted, and the detection module 1410 having the magnets 1413 mounted thereto will be described in detail.

In the test device 1000 according to the illustrated embodiment, the detection module 1410 may include at least one magnet 1413, as shown in FIG. 23.

The magnet 1413 provided in the detection module 1410 applies attractive force to the magnetic body 161 of the magnetic body accommodating chamber 160 formed in a region adjacent to the detection object 20, in order to identify the position of the detection object 20 of the microfluidic device 10. In the illustrated embodiment, a magnet is accommodated in the detection module, and a magnetic material is accommodated in the magnetic body accommodating chamber of the microfluidic device. However, embodiments of the present invention are not limited thereto. The magnetic material may be accommodated in the detection module, and the magnet may be accommodated in the magnetic body accommodating chamber of the microfluidic device.

When the magnet 1413 of the detection module 1410 and the magnetic body 161 of the microfluidic device 10 face each other, attractive force is applied to the magnetic body 161 by the magnet 1413, and the microfluidic device 10 may remain in place as long as force exceeding the attractive force is not applied thereto.

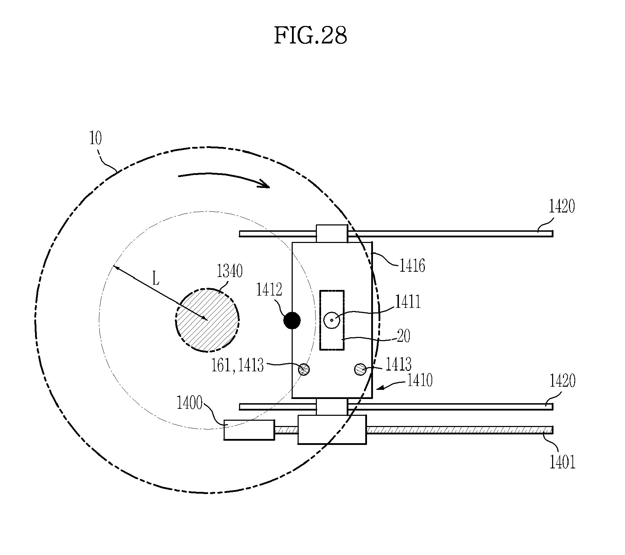

As shown in FIG. 28, when the magnetic body 161 of the microfluidic device 10 is positioned to face the magnet 1413 and the position thereof is fixed by the attractive force, the magnet 1413 in the detection module 1410 is arranged at a position at which the detection object 20 faces the light receiving element 1411 of the detection module 1410.

That is, when the magnetic body 161 of the microfluidic device 10 and the magnet 1413 of the detection module 1410 are arranged to face each other, the detection object 20 naturally faces the light receiving element 1411.

As such, with the magnet 1413 installed at the detection module 1410, when the magnetic body 161 is moved close to the magnet 1413 by rotation of the microfluidic device 10 toward the light receiving element 1411 to detect the detection object 20, the magnetic body 161 is fixed by the attractive force of the magnet 1413, and thereby the microfluidic device 10 stops moving, with the detection object 20 facing the light receiving element 1411.

Accordingly, the detection object 20 may be stably detected. Shock applied to the detection object 20 from the outside during detection of the detection object 20 may interfere with accurate detection of the detection object 20. In this case, stable detection of the detection object 20 may be ensured by the attractive force acting between the magnet 1413 and the magnetic body 161 to stably fix the microfluidic device 10.

In FIGS. 23 to 28, the detection module 1410 is provided with two magnets 1413. However, the number of the magnets is not limited thereto since the magnetic body 161 is moveable to the position facing the magnet 1413 of the detection module 1410 from any position on the microfluidic device 10, through movement of the detection module 1410 in a radial direction and rotation of the microfluidic device 10.

In the illustrated embodiment, the detection module 1410 is provided with two magnets 1413 since two or more magnets 1413 allow other detection objects 20 at different radii to move a shorter distance and face the light receiving element 1411 when detection is to be performed than when only one magnet 1413 is provided.

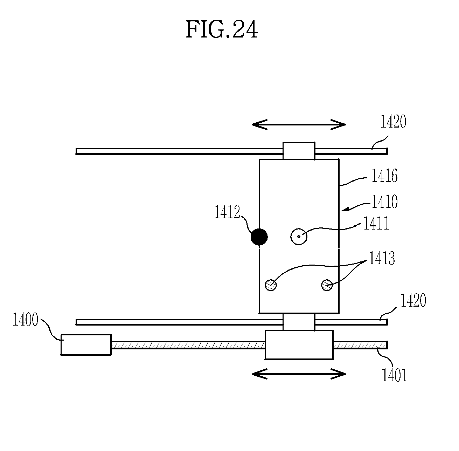

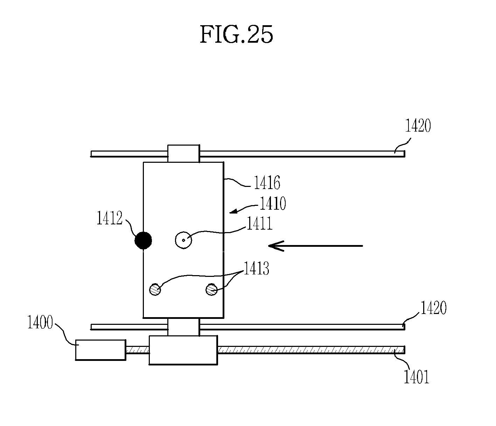

FIGS. 24 and 25 are views illustrating a radial movement of a detection module 1410 according to another embodiment, in which the detection module 1410 is viewed from the top.

The configuration and operation of the detection module 1410 is the same as the detection module 1410 of FIGS. 17 and 18 except for the magnets 1413 which are additionally installed, and thus a description thereof will be omitted.

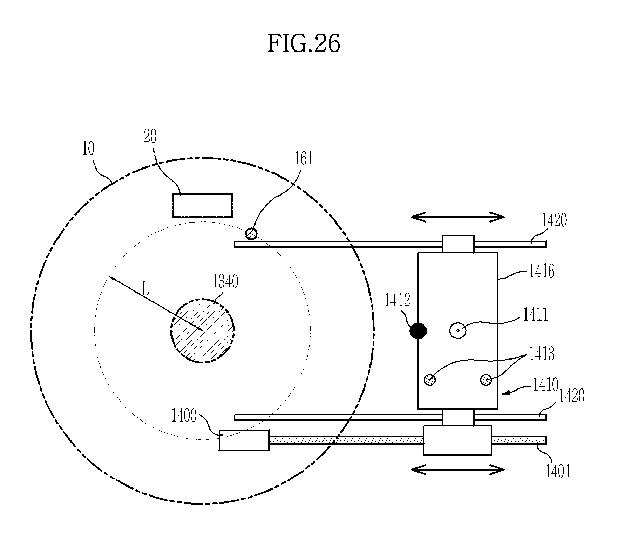

FIGS. 26 to 28 are views illustrating movement of a detection object 20 to a position facing the light receiving element 1411 of the detection module 1410 by movement of the detection module 1410 and rotation of the microfluidic device 10 in a test device 1000 according to another embodiment of the present invention.

The microfluidic device 10, which is positioned at the upper side of the detection module 1410, is shown in a dotted line to more clearly show movement of the detection module 1410, which is positioned at the lower side of the microfluidic device 10 and shown in a solid line.

FIG. 26 shows the microfluidic device 10 which has stopped rotating as the reaction in the microfluidic device 10 is completed. In FIG. 26, the detection module 1410 is positioned below the microfluidic device 10, beyond the outer circumference of the microfluidic device 10. For convenience of description, other structures in the microfluidic device 10 are not shown, and only one detection object 20 and one magnetic body 161 to identify the position of the detection object 20 are shown.

When the reaction in the microfluidic device 10 is completed and rotation of the microfluidic device 10 is stopped, the controller 1200 controls the detection module drive unit 1400 to move the detection module 1410 in a radial direction such that one of the magnets 1413 of the detection module 1410 is positioned at a radius L at which the magnetic body 161 installed in a region adjacent to the detection object 20 to be detected is positioned.

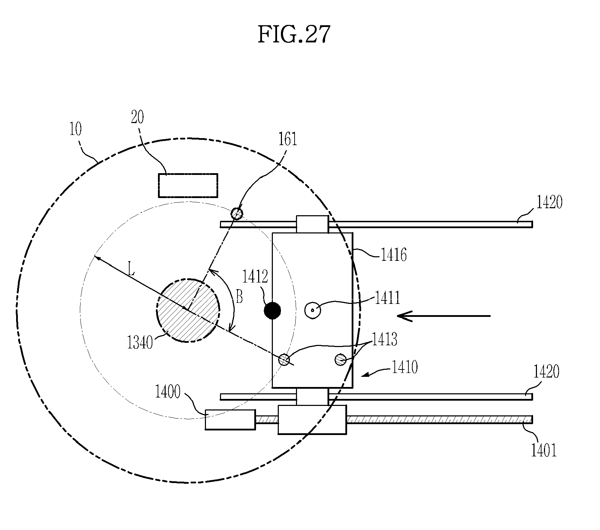

FIG. 27 illustrates that the magnet 1413 of the detection module 1410 has reached radius L at which the magnetic body 161 of the microfluidic device 10 is installed according to movement of the detection module 1410 toward the center of the microfluidic device 10 in a radial direction.

Information on radii at which the magnetic bodies 161 provided in the microfluidic device 10 are installed may be pre-stored in the controller 1200. Upon actuating the detection module drive unit 1400, the controller 1200 controls the detection module drive unit 1400 using the information such that the magnet 1413 of the detection module 1410 is moved to a position corresponding to radius L at which the magnetic body 161 arranged in a region adjacent to a detection object 20 to be detected is positioned.

When the magnet 1413 of the detection module 1410 is positioned at radius L at which the magnetic body 161 of the microfluidic device 10 is positioned, the controller 1200 controls the rotary drive unit 1340 to rotate the microfluidic device 10 such that the magnetic body 161 of the microfluidic device 10 is moved to a position facing the magnet 1413 of the detection module 1410.

When the magnetic body 161 approaches the magnet 1413 of the detection module 1410, the magnetic body 161 is fixed at the position facing the magnet 1413 by the attractive force between the magnet 1413 and the magnetic body 161. Thereby, the microfluidic device 10 stops with the detection object 20 of the microfluidic device 10 facing the light receiving element 1411 of the detection module 1410.

FIG. 28 shows the magnetic body 161 of the microfluidic device 10 moved to the position facing the magnet 1413 of the detection module 1410 by rotation of the microfluidic device 10 and fixed in place by the attractive force of the magnet 1413 applied thereto.

The controller 1200 may calculate an angle B between the magnetic body 161 of the microfluidic device 10 and the magnet 1413 of the detection module 1410 and control the rotary drive unit 1340 to rotate the microfluidic device 10 by the angle

B.

When the magnetic body 161 of the microfluidic device 10 moves to the position facing the magnet 1413 of the detection module 1410 and the microfluidic device 10 stops, the light emitting element 1333 is controlled by the controller 1200 to emit light to the detection object 20, and the light receiving element 1411 creates an image of the detection object 20 using the light emitted from the light emitting element 1333 and transmitted through the detection object 20.

The controller 1200 computes presence or concentration of the analyte by analyzing the created image of the detection object 20.

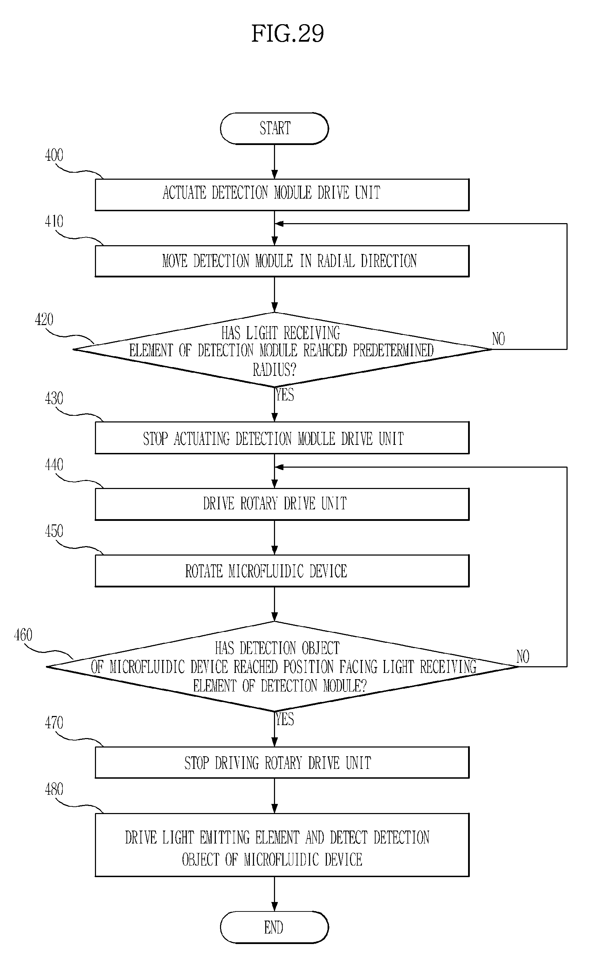

FIG. 29 is a flowchart illustrating a control method of the test device 1000 according to one embodiment of the present invention.

Referring to FIG. 29, the controller 1200 first actuates the detection module drive unit 1400 (400), and moves the detection module 1410 in a radial direction (410).

When the reaction in the microfluidic device 10 is completed and rotation of the microfluidic device 10 is stopped, the controller 1200 controls the detection module drive unit 1400 to move the detection module 1410 in a radial direction such that the light receiving element 1411 of the detection module 1410 is moved to a position at the radius at which the detection object 20 to be detected is installed.

The controller 1200 determines whether the light receiving element 1411 of the detection module 1410 has reached the predetermined radius (420), and if the light receiving element 1411 of the detection module 1410 has reached the predetermined radius, the controller 1200 stops actuation of the detection module drive unit 1400 (430).

Here, the predetermined radius represents a radius R on the microfluidic device 10 at which the detection object 20 provided in the microfluidic device 10 is positioned. Information on radii at which the detection objects 20 provided in the microfluidic device 10 are positioned may be pre-stored in the controller 1200. Upon actuating the detection module drive unit 1400, the controller 1200 controls the detection module drive unit 1400 using the information such that the light receiving element 1411 of the detection module 1410 is moved to the radius R at which a detection object 20 to be detected is positioned.

When the light receiving element 1411 of the detection module 1410 is positioned at radius R at which the detection object 20 of the microfluidic device 10 is positioned and the detection module drive unit 1400 stops, the controller 1200 actuates the rotary drive unit 1340 (440) and rotates the microfluidic device 10 (450).

That is, the controller 1200 controls the rotary drive unit 1340 to rotate the microfluidic device 10 such that the detection object 20 of the microfluidic device 10 is moved to a position facing the light receiving element 1411 of the detection module 1410.

The controller 1200 calculates an angle A between the detection object 20 of the microfluidic device 10 and the light receiving element 1411 of the detection module 1410 and controls the rotary drive unit 1340 to rotate the microfluidic device 10 by the angle A.

The controller 1200 determines whether the detection object 20 of the microfluidic device 10 has reached the position facing the light receiving element 1411 of the detection module 1410 (460), and if the detection object 20 has reached the position facing the light receiving element 1411 of the detection module 1410, the controller 1200 stops actuation of the rotary drive unit 1340 (470).

When the detection object 20 reaches the position facing the light receiving element 1411 of the detection module 1410 and the controller 1200 stops actuating the rotary drive unit 1340, the microfluidic device 10 stops moving, with the detection object 20 of the microfluidic device 10 facing the light receiving element 1411 of the detection module 1410.

When the detection object 20 of the microfluidic device 10 reaches the position facing the light receiving element 1411 of the detection module 1410 and the microfluidic device 10 stops rotating as actuation of the rotary drive unit 1340 is stopped, the controller 1200 actuates the light emitting element 1333 to detect the detection object 20 of the microfluidic device 10 (480).

The controller 1200 drives the light emitting element 1333 to emit light to the detection object 20, and the light receiving element 1411 creates an image of the detection object 20 using the light emitted from the light emitting element 1333 and transmitted through the detection object 20. The controller 1200 computes presence or concentration of the analyte by analyzing the created image of the detection object 20.

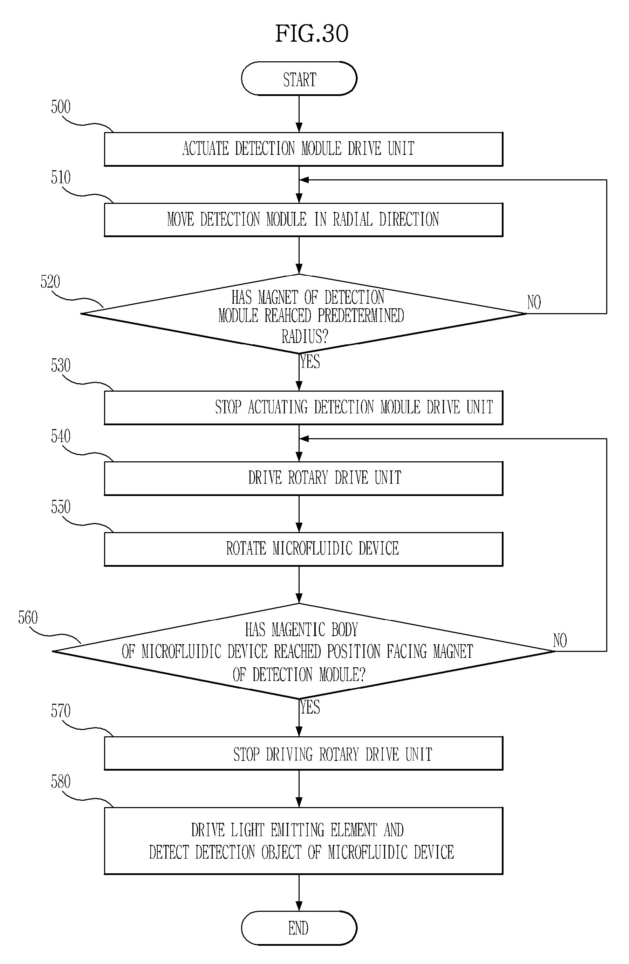

FIG. 30 is a flowchart illustrating a control method of the test device 1000 according to another embodiment of the present invention.

Referring to FIG. 30, the controller 1200 first actuates the detection module drive unit 1400 (500), and moves the detection module 1410 in a radial direction (510).

When the reaction in the microfluidic device 10 is completed and rotation of the microfluidic device 10 is stopped, the controller 1200 controls the detection module drive unit 1400 to move the detection module 1410 in a radial direction such that one of the magnets 1413 of the detection module 1410 is positioned at radius L at which the magnetic body 161 installed in a region adjacent to the detection object 20 to be detected is positioned.

The controller 1200 determines whether the magnet 1413 of the detection module 1410 has reached the predetermined radius (520), and if the magnet 1413 of the detection module 1410 has reached the predetermined radius, the controller 1200 stops actuation of the detection module drive unit 1400 (530).

Here, the predetermined radius is a radius L on the microfluidic device 10 at which the magnetic body 161 provided in the microfluidic device 10 is installed. Information on radii at which the magnetic bodies 161 provided in the microfluidic device 10 are installed may be pre-stored in the controller 1200. When actuating the detection module drive unit 1400, the controller 1200 controls the detection module drive unit 1400 using the information such that the magnet 1413 of the detection module 1410 is moved to a position corresponding to the radius L at which the magnetic body 161 arranged in a region adjacent to a detection object 20 to be detected is positioned.

When the magnet 1413 of the detection module 1410 is positioned at radius L at which the magnetic body 161 of the microfluidic device 10 is positioned and the detection module drive unit 1400 stops, the controller 1200 actuates the rotary drive unit 1340 (540) and rotates the microfluidic device 10 (550).

That is, the controller 1200 controls the rotary drive unit 1340 to rotate the microfluidic device 10 such that the magnetic body 161 of the microfluidic device 10 is moved to the position facing the magnet 1413 of the detection module 1410. The controller 1200 may calculate the angle B between the magnetic body 161 of the microfluidic device 10 and the magnet 1413 of the detection module 1410 and control the rotary drive unit 1340 to rotate the microfluidic device 10 by the angle B.

The controller 1200 determines whether magnetic body 161 of the microfluidic device 10 has reached the position facing the magnet 1413 of the detection module 1410 (560), and if the magnetic body 161 has reached the position facing the magnet 1413 of the detection module 1410, the controller 1200 stops actuation of the rotary drive unit 1340 (570).

When the magnetic body 161 reaches the position facing the magnet 1413 of the detection module 1410 and the controller 1200 stops actuating the rotary drive unit 1340, the magnetic body 161 is fixed at the position facing the magnet 1413 by attractive force between the magnet 1413 and the magnetic body 161. Thereby, the microfluidic device 10 stops with the detection object 20 of the microfluidic device 10 facing the light receiving element 1411 of the detection module 1410.

When the magnetic body 161 of the microfluidic device 10 reaches the position facing the magnet 1413 of the detection module 1410 and actuation of the rotary drive unit 1340 is stopped to stop rotation of the microfluidic device 10, the controller 1200 drives the light emitting element 1333 to detect the detection object 20 of the microfluidic device 10 (580).

The light emitting element 1333 is controlled by the controller 1200 to emit light to the detection object 20, and the light receiving element 1411 creates an image of the detection object 20 using the light emitted from the light emitting element 1333 and transmitted through the detection object 20. The controller 1200 computes presence or concentration of the analyte by analyzing the created image of the detection object 20.

### <Test Device/Method of Suppressing Brightness Variation among Images of the Detection Object>

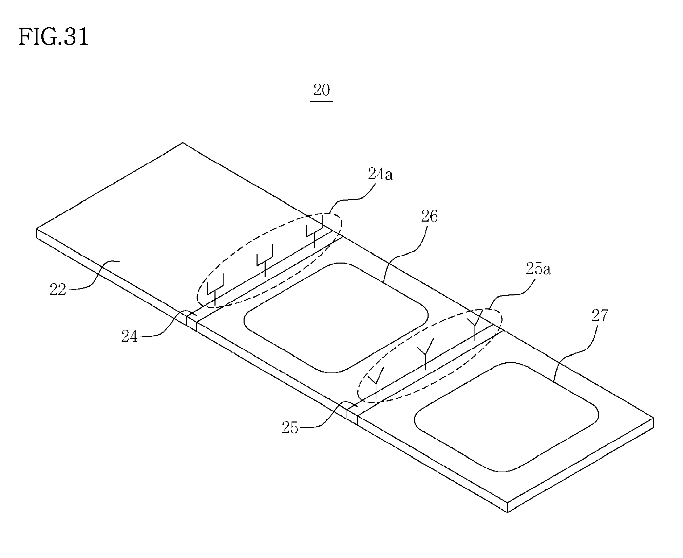

FIG. 31 is a view schematically illustrating test paper used as an example of the detection object of a test device according to one embodiment of the present invention.

The detection object 20 includes a test paper-type object formed of a micropore, micro pillar, or thin porous membrane such as cellulose and allowing capillary pressure to act. Referring to FIG. 31, a sample pad 22 to which the sample is applied is formed at one end of the detection object 20, and a test line 24 on which the first capture material 24a to detect the analyte is immobilized is formed at one end of the sample pad 22 facing the other end of the detection object 20.

When a biosample such as blood or urine is dropped on the sample pad 22, the biosample flows to the opposite side due to the capillary pressure. For example, if the analyte is antigen Q and binding between the analyte and the first marker conjugate occurs in the second chamber 130, the biosample contains a conjugate of antigen Q and the first marker conjugate.

In case that the analyte is antigen Q, the capture material 24a immobilized on the test line 24 may be antibody Q. When the biosample flowing according to the capillary pressure reaches the test line 24, the conjugate of antigen Q-the first marker conjugate binds with antibody Q 24a to form a sandwich conjugate. Therefore, if the analyte is contained in the biosample, it may be detected by the marker on the test line 24.

If the amount of the sample is small or the sample is contaminated, a normal test often fails for various reasons. Accordingly, to determine whether the test has been normally performed, the detection object 20 is provided with a control line 25 on which a second capture material 25a specifically reacting with a specific material contained in the sample regardless of presence of the analyte is immobilized. As described above in FIG. 2, the sample that has passed the second chamber 130 contains the second marker conjugate having a certain material which specifically reacts with the second capture material 25a, and therefore, if the sample has normally flowed on the detection object 20, the second marker conjugate will bind with the second capture material 25a on the control line 25 and the binding will be marked by the marker.

As the second capture material 25a immobilized on the control line 25, biotin may be used, and thereby the second marker conjugate in the sample in the second chamber 130 is a streptavidin-marker conjugate which has a high affinity to biotin.

If the sample is normally moved to the opposite side by osmotic pressure, the second marker conjugate is also moved along with the sample. Accordingly, regardless of presence of the analyte in the sample, a conjugate is formed by conjugation between the second marker conjugate and the second capture material 25a, and is marked on the control line 25 by the maker.

That is, if a mark by the marker appears on both the control line 25 and the test line 24, the sample will be deemed positive, which indicates that the analyte is present in the sample. If the mark appears only on the control line 25, the sample will be deemed negative, which indicates that the analyte is not present in the sample. On the contrary, if the mark does not appear on the control line 25, it may be determined that the test has not been normally performed.

Meanwhile, the light receiving element 1411 captures an image of the test paper described above through the light emitted from the light emitting element 1333. A signal value of the test line 24 is obtained from the captured image of the test paper to detect the presence or concentration of the analyte.

Meanwhile, depending on the condition of the test paper, brightness may vary among images captured. This may lead to variation in assay results, and uniform brightness of the test paper may need to be maintained.

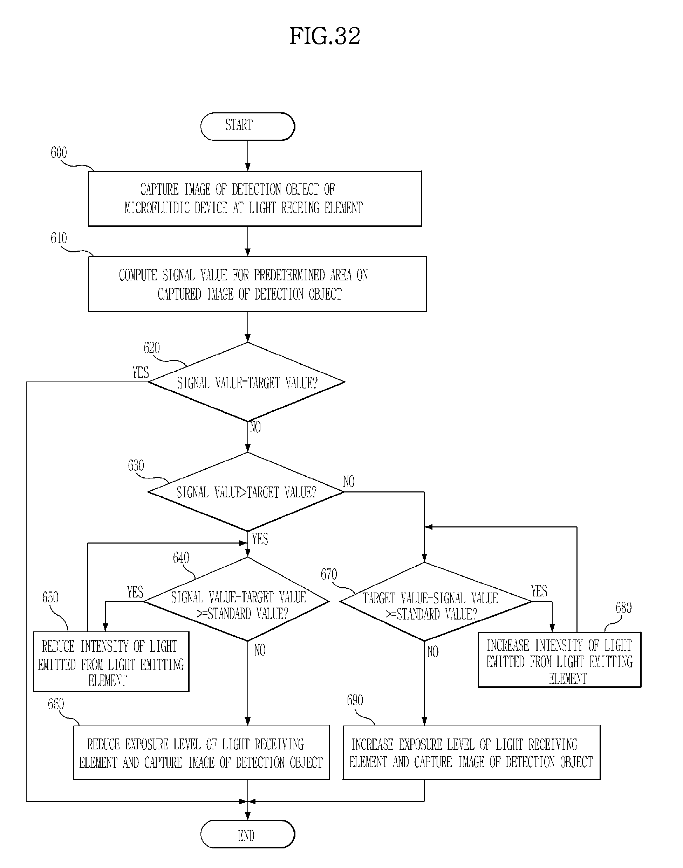

FIG. 32 is a flowchart illustrating a control method of a test device 1000 according to an embodiment of the present invention to capture an image of the detection object 20, i.e., test paper, with a uniform brightness of the test paper maintained in the test device 1000.

Referring to FIG. 32, the light receiving element 1411 first captures an image of the detection object 20 of the microfluidic device 10, i.e., test paper (600). The light receiving element 1411 transmits the captured image of the test paper to the controller 1200.

The controller 1200 computes a signal value for a predetermined area of the detection object 20 (610).

That is, the controller 1200 computes a signal value for the predetermined area from the image of the test paper transmitted from the light receiving element 1411.

Here, the predetermined area may include, as shown in FIG. 31, an area between the test line 24 and the control line 25 (a first area 26) or an area on the right side of the control line 25 (a second area 27).

Embodiments of the present invention are not limited thereto, and a signal value may be computed for a different area on the test paper. However, the signal values of the test line 24 and the control line 25 are important for analysis of the reaction result, and thus a signal value may be computed for the area between the test line 24 and the control line 25 or the area on the right side of the control line 25.

Here, as the signal value, an RGB signal value, a YCbCr signal value or a Gray-scale signal value may be used.

After the signal value is computed, the controller 1200 determines whether the computed signal value is equal to a predetermined target value (620). Here, one of the signal value of the first region 26 and the signal value of the second region 27 may be compared with a target value, or an average of the signal values of the first region 26 and the second region 27 may be compared with the target value. Hereinafter, a description will be given of a case in which the signal value of the first region 26 is compared with the target value.

Here, the target value, which is a value that the computed signal value should comply with, reflects the optimum brightness targeted in capturing an image of the test paper. The target value may be determined through repeated experimentation and pre-stored in the controller 1200.

The controller 1200 compares the computed signal value with the target value, and depending on the result of comparison, controls the light receiving element 1411 or the light emitting element 1333 to adjust the brightness of the test paper to ensure that an image of the test paper is captured with a uniform brightness.

If the signal value is equal to the target value, the control operation is terminated since separate adjustment of brightness is unnecessary.

If the signal value is different from the target value, the controller 1200 determines if the signal value exceeds the target value (630). If the signal value exceeds the target value, the controller 1200 determines that the brightness of the test paper is higher than the optimum brightness and performs a control operation to capture a darker image of the test paper.

That is, if the signal value is greater than the target value, the controller 1200 determines whether the difference between the signal value and the target value is greater than or equal to a predetermined standard value (640).

If the difference between the signal value and the target value is greater than or equal to the predetermined standard value, the controller 1200 controls the light emitting element 1333 to lower the intensity of light emitted from the light emitting element 1333 (650).

When the difference between the signal value and the target value is lowered below the predetermined standard value through this operation, the controller 1200 reduces the exposure level of the light receiving element 1411 to capture a darker image of the detection object 20 (660).

If the signal value is greater than the target value, this indicates that the brightness of the test paper is higher than the optimum brightness as defined by the target value and thus, to obtain a detection result having low brightness variation, a darker image of the test paper may need to be captured.

If the signal value is greater than the target value and the difference therebetween is greater than or equal to the predetermined standard value, the controller 1200 determines that the difference between the signal value and the target value is large.

That is, the controller 1200 determines that a control operation needs to be performed to roughly reduce the intensity of light emitted from the light emitting element 1333 to a certain level prior to fine adjustment of the brightness through control of the exposure level of the light receiving element 1411.

Here, the standard value is a value defining a difference between the signal value and the target value by which the light intensity of the light emitting element 1333 needs to be reduced prior to adjustment of the brightness of an image of the test paper though control of the exposure level of the light receiving element 1411. The standard value may be determined through repeated experimentation and pre-stored in the controller 1200.

As described above, the controller 1200 reduces the intensity of light emitted from the light emitting element 1333 to a certain level if the signal value is greater than the target value and the difference therebetween is greater than or equal to the standard value and thereby, when the difference between the signal value and the target value is below the standard value, the controller 1200 reduces the exposure level of the light receiving element 1411 and captures an image of the detection object 20.

The controller 1200 controls at least one of shutter speed and iris opening of the light receiving element 1411 to reduce the exposure level of the light receiving element 1411, i.e., to reduce the intensity of light received from the light receiving element 1411 to capture an image of the detection object 20.

The relationship between variation in the exposure level of the light receiving element 1411 and change in the signal value may be pre-established and pre-stored in the controller 1200, and the controller 1200 computes, with reference to this relationship, an exposure level of the light receiving element 1411 at which the signal value is set to have the target value that it should meet, and accordingly reduces the exposure level of the light receiving element 1411.

In operation 630, if the signal value is less than the target value, the controller 1200 determines that the brightness of the test paper is lower than the optimum brightness and performs a control operation to capture a brighter image of the test paper.

That is, if the signal value is less than the target value, the controller 1200 determines whether the difference between the target value and the signal value is greater than or equal to the predetermined standard value (670).

If the difference between the target value and the signal value is greater than or equal to the predetermined standard value, the controller 1200 controls the light emitting element 1333 to increase the intensity of light emitted by the light emitting element 1333 (680).

Thereby, when the difference between the target value and the signal value is below the predetermined standard value, the controller 1200 increases the exposure level of the light receiving element 1411 and captures a brighter image of the detection object 20 (690).

If the signal value is less than the target value, this indicates that the brightness of the test paper is lower than the optimum brightness reflected in the target value, and thus to obtain a detection result having a low brightness variation, a brighter image of the test paper may need to be captured.

If the signal value is less than the target value, and the difference between the target value and the signal value is greater than or equal to the predetermined standard value, the controller 1200 determines that the difference between the signal value and the target value is large.

That is, the controller 1200 determines that a control operation needs to be performed to roughly increase the intensity of light emitted from the light emitting element 1333 to a certain level prior to fine adjustment of the brightness through control of the exposure level of the light receiving element 1411.

Here, the standard value is a value defining a difference between the signal value and the target value by which the light intensity of the light emitting element 1333 needs to be increased prior to adjustment of the brightness of an image of the test paper though control of the exposure level of the light receiving element 1411. The standard value may be determined through repeated experimentation and pre-stored in the controller 1200. The standard value of operation 670 may be set to be equal to that of operation 640.

As described above, the controller 1200 increases the intensity of light emitted from the light emitting element 1333 to a certain level if the signal value is less than the target value and the difference therebetween is greater than or equal to the standard value and thereby, when the difference between the target value and the signal value is below the standard value, the controller 1200 increases the exposure level of the light receiving element 1411 and captures an image of the detection object 20.

The controller 1200 controls at least one of shutter speed and iris opening of the light receiving element 1411 to increase the exposure level of the light receiving element 1411, i.e., to increase the intensity of light received from the light receiving element 1411 to capture an image of the detection object 20.

The relationship between variation in the exposure level of the light receiving element 1411 and change in the signal value may be pre-established and pre-stored in the controller 1200, and the controller 1200 computes, with reference to this relationship, an exposure level of the light receiving element 1411 at which the signal value is set to have the target value that it should meet, and accordingly increases the exposure level of the light receiving element 1411.

As is apparent from the above description, abnormal seating of the microfluidic device on the rotary drive unit and the clamper may be prevented, and therefore a test error may be prevented from occurring due to failure of rotation of the microfluidic device.

Although a few embodiments of the present invention have been shown and described, it would be appreciated by those skilled in the art that changes in form and details may be made in these embodiments without departing from the principles and spirit of the invention, the scope of which is defined in the claims and their equivalents.

## Claims

1. A control method of a test device (1000) **characterized by**:
mounting a microfluidic device (10) on a tray (1320);
upon mounting the microfluidic device on the tray, positioning a rotary drive unit (1340) at a center of the microfluidic device; and
moving the rotary drive unit up and down at least once.

2. The control method according to claim 1, **characterized in that**, in moving the rotary drive unit up and down at least once, the center of the microfluidic device is coupled to a clamper (1330) positioned at a housing (1010).

3. The control method according to claim 1 or 2, **characterized in that** the rotary drive unit is coupled to a vertical movement unit (1310) and substantially moves up and down by vertical movement.

4. The control method according to claim 3, **characterized in that** the center of the microfluidic device is seated at a clamper (1330) positioned at an upper housing (1012) by vertical movement of the vertical movement unit.

5. The control method according to claim 3 or 4, **characterized in that**, in mounting the microfluidic device on the tray, the tray moves to a first state,
wherein the tray is positioned at an exterior of a housing to allow the microfluidic device to be loaded onto the tray, and
upon loading the microfluidic device onto the tray, the tray moves to a second state positioned at an interior of the housing.

6. The control method according to claim 5, **characterized in that**, in the second state, the vertical movement unit performs a first movement of moving upward and a second movement of moving downward at least once,
the microfluidic device is coupled to the clamper by the first movement, and the second movement is performed to secure the microfluidic device to the rotary drive unit.

7. The control method according to any of claims 2-6, **characterized in that**, upon closing the tray, moving a detection module (1410), including a magnet (1413), at least once, and seating the microfluidic device,
wherein the detection module repeats a third movement of moving to one side in the housing and a fourth movement of moving in a direction opposite to the third movement at least once.

8. The control method according to claim 7, further comprising, moving the detection module to an outermost circumference of the housing, upon coupling the microfluidic device to the clamper.

9. A test device (1000) **characterized by**:
a housing (1010) forming an external appearance of the test device;
a tray (1320);
a microfluidic device (10) being loaded onto the tray;
a rotary drive unit (1340) coupled to a center of the microfluidic device and adapted to move in a first movement toward a position at which the tray is placed and a second movement in a direction opposite to the first movement;
an opening-closing drive unit (1300) configured to move the rotary drive unit to perform the first movement and the second movement at least once; and
a controller (1200) configured to control the opening-closing drive unit to initiate the rotary drive unit to perform the first movement and the second movement at least once when the microfluidic device is loaded onto the tray such that the microfluidic device is accommodated at a predetermined position.

10. The test device (1000) according to claim 9, **characterized in that** the rotary drive unit is coupled to a vertical movement unit (1310) positioned inside the housing, and the vertical movement unit adapted to perform the first movement and the second movement at least once.

11. The test device (1000) according to claim 9 or 10, **characterized in that** the housing includes further comprises an upper housing (1012) arranged at an upper side of the tray, a lower arranged at a lower side of the tray, and a clamper (1330) to seat the microfluidic device on the tray , wherein the clamper is arranged at an interior of the upper housing.

12. The test device (1000) according to claim 9, 10 or 11, **characterized in that** the controller is configured to control the tray to move to a first state, wherein the tray is positioned at an exterior of the housing and to a second state, wherein the tray is positioned at an interior of the housing.

13. The test device (1000) according to claim 12, **characterized in that** movement of the tray and movement of the vertical movement unit are implemented by the opening-closing drive unit,
wherein the controller is configured to control the second movement of the vertical movement unit such that the tray is not opened.

14. The test device (1000) according to any of claims 9-13, further comprising a detection module (1410) including a light emitting element (1333) adapted to emit light to the microfluidic device, and a light receiving element (1411) adapted to receive light emitted from the light emitting element (1333) and transmitted through the microfluidic device, wherein the light emitting element (1333) is arranged at at least one portion of the housing(1010).

15. The test device (1000) according to claim 14, further comprising a detection module drive unit (1400) to drive the detection module,
wherein the detection module is adapted to perform a third movement of moving to one side in the housing and a fourth movement of moving in a direction opposite to the third movement,
wherein the controller is configured to control the detection module drive unit to initiate the detection module to perform the third movement and the fourth movement at least once such that the microfluidic device is accommodated at a predetermined position.
